# EUROPEAN PATENT SPECIFICATION

(11) **EP 4 463 162 B1**
(45) Date of publication and mention of the grant of the patent: **22.07.2026**
(21) Application number: 23700741.4
(22) Date of filing: 11.01.2023
(51) Int. Cl.: A61K 38/17, A61K 31/502, A61P 35/00

(54) **CONNEXIN 43 FOR USE IN TREATING BRCA1/2-MUTATED CANCER**
CONNEXIN 43 ZUR VERWENDUNG BEI DER BEHANDLUNG VON BRCA1/2-MUTIERTEM KREBS
CONNEXINE 43 POUR SON UTILISATION DANS LE TRAITEMENT D'UN CANCER PORTEUR D'UNE MUTATION BRCA1/2

(30) Priority: 11.01.2022 EP 22382009
(43) Date of publication of application: 20.11.2024
(73) Proprietor: Fundación Profesor Novoa Santos, 15006 A Coruña (ES); Servizo Galego de Saúde (SERGAS), 15703 Santiago de Composteia (ES)
(72) Inventor: MAYÁN SANTOS, María Dolores, 15006 A Coruña (ES); RODRÍGUEZ-CANDELA MATEOS, Marina, 15006 A Coruña (ES); ACEA NEBRIL, Benigno, 15703 Santiago de Compostela (A Coruña) (ES); SANTIAGO FREIJANES, María Paz, 15703 Santiago de Compostela (A Coruña) (ES)
(74) Representative: Hoffmann Eitle
(86) International application number: PCT/EP2023/050542
(87) International publication number: WO 2023/135169

(56) References cited:
- WO-A1-2014/191608
- WO-A1-2017/031442
- WO-A1-2019/213217
- WO-A1-2020/142583
- SABIT HUSSEIN ET AL: "Triple negative breast cancer in the era of miRNA", CRITICAL REVIEWS IN ONCOLOGY/HEMATOLOGY, ELSEVIER, AMSTERDAM, NL, vol. 157, 9 December 2020 (2020-12-09), XP086454867, ISSN: 1040-8428, [retrieved on 20201209], DOI: 10.1016/J.CRITREVONC.2020.103196

## Description

### FIELD OF THE INVENTION

The present invention refers to the medical field. Particularly, the present invention refers to Connexin 43 (Cx43) for use in the treatment of *BRCA1*/*2* mutated cancer selected from: *BRCA1*/*2* mutated triple negative breast cancer (TNBC) and/or *BRCA1*/*2* mutated ovarian cancer and/or *BRCA1*/*2* mutated lung cancer; preferably in combination with a poly ADP ribose polymerase (PARP) inhibitor (PARPi).

### STATE OF THE ART

*BRCA1*/*2 (BRCA1* and *BRCA2)* genes code for tumour suppressor proteins involved in DNA repair. Deleterious mutations in these genes increase breast and ovarian cancer risk 5 and 10-30 times the normal numbers, respectively. Although only 5-10 % breast cancer cases are due to these mutations, their impact is far superior through the high risk they are associated to. Between 50-65 % *BRCA1*-mutated women will develop breast cancer by age 70, and between 35-46 % in the case of ovarian cancer; between 40-57 % women with mutations in *BRCA2* will suffer mammary neoplasia by age 70, and between 13-23 % will develop ovarian carcinoma. The vast majority of *BRCA* mutated breast tumours belong to the triple negative breast cancer subtype. In line with this, the third most common cancer associated to mutations in *BRCA1*/*2* is pancreatic carcinoma, and these germline mutations significantly raise the lifetime risk of this cancer. Overall relative risk of pancreatic cancer among *BRCA1* and *BRCA2* mutation carriers is around 2.26 and 2-9, respectively, and it has been estimated that 6-10 % of all pancreatic adenocarcinoma patients carry a germline or a somatic *BRCA1*/*2* mutation. On the other hand, *BRCA2* germline mutations are the genetic factors that lead to the highest risk of prostate cancer (8.6-fold in men ≤ 65 years), the second most common cancer in men worldwide. Although less common, *BRCA1* mutations (1-2 % versus 0.44 %) are also associated to higher risk of sporadic prostate neoplasm (3.5-fold), and deleterious germline mutations in both genes are linked to poor outcome and higher aggressiveness. Finally, regarding lung cancer, a *BRCA2* mutation (c.9976T) has been associated to 85 % higher probability of presenting any type of lung cancer, and smokers harboring that specific mutation are at almost twice (25 %) the overall risk of developing lung cancer.

Recent investigations have turned their attention to the therapeutic chance of "exploiting" this type of mutated cancer to eliminate cancer cells, taking advantage of its lack of *BRCA1*/*2* protein/s. The mechanism of action of this strategy is based on the inhibition of the other main enzymes involved in DNA repair, the poly (ADP-ribose) polymerases (PARPs) (PARP inhibition, PARPi). The so-called PARP inhibitors represent a targeted treatment that acts by blocking the DNA repair response in tumours deficient in the homologous recombination repair process (HRR), such as *BRCA1*/*2* mutated cells. These compounds inhibit PARPs and trap them at DNA single-strand breaks (SSBs), eventually leading to double-strand breaks (DSBs) and cell death in the absence of other repairing agents in a process called synthetic lethality. PARPi have been able to increase progression-free survival (metastatic carcinomas) and have also induced remarkable tumour shrinkage in *BRCA1*/*2* mutated breast, ovarian, pancreatic and prostate cancer. Recent research has also set the focus on *BRCA1*/*2* mutated lung adenocarcinoma, as treatment with PARPi has shown promising results, particularly in small cell lung cancer.

Among the available PARP inhibitors (olaparib, niraparib, veliparib, talazoparib, rucaparib), olaparib (*AZD-2281, Lynparza,* AstraZeneca) is one of the most efficient, and has been recently authorized in the United States by the Food and Drug Administration and in the European Union by the European Medicines Agency (ref. EMEA/H/C/003726) for the treatment of the following neoplasias, both in monotherapy and also in combination with bevacizumab:
- High-grade cancer of the ovaries, fallopian tubes and peritoneum with or without mutations in *BRCA1*/*2,* previously treated efficiently with specific chemotherapy, and which may have relapsed.
   ▪ HER2-negative *BRCA1*/*2*-mutated breast cancer that has spread beyond the initial source and for which specific chemotherapies have not been effective.
   ▪ Metastatic *BRCA1*/*2*-mutated pancreatic cancer that has not grown worse after at least 4 months of platinum-based chemotherapy.
   ▪ Metastatic *BRCA1*/*2*-mutated prostate cancer after unsuccessful surgical and hormono-/chemo-therapeutic treatment.

Although olaparib has been more implemented in the context of ovarian cancer, increasing evidence supports its use as a monotherapy or maintenance therapy in triple negative *BRCA1*/*2*-mutated breast cancer, such as the clinical trials OlympiAD (ClinicalTrials.gov Identifier NCT02032823), PETREMAC (ClinicalTrials.gov identifier NCT02624973) or ICEBERG1 (ClinicalTrials.gov Identifier NCT00494234).

In spite of the undeniable attractiveness of this therapeutic approach for *BRCA1*/*2*-mutated cancer, there are diverse clinical phenotypes with *de novo* resistance to PARPi, and only 20-40 % of potentially suitable patients do benefit from it. Among the different mechanisms involved in PARPi resistance, it is important to highlight higher drug efflux, restoration of the homologous recombination repair process (HRR), disruption of PARP1 and PARG proteins and restitution of the replication forks stability. Regarding preclinical evidence of PARPi resistance, the HCC1937 TNBC cell line, which is homozygous for the *BRCA1* 5382insC mutation, has frequently been reported as being relatively olaparib resistant, in relation with a potential preservation of DNA repair pathways, representing an appropriate candidate to study *de novo* resistance to olaparib in *BRCA1* mutated TNBC.

Due to the high heterogeneity of triple negative breast cancer and the lack of therapeutic targets, as well as the associated clinical risk of *BRCA1*/*2* mutations in ovarian, pancreatic, prostate and lung cancer, it is crucial to exploit *BRCA1*/*2*-mutated tumours singular characteristic/weakness and try to evade resistance to PARPi.

So, there is an unmet medical need of finding strategies aimed at improving sensitivity and reducing resistance to PARPi in *BRCA1*/*2* mutated tumours (e.g., breast, ovarian, pancreatic, prostate and lung cancer). The present invention is focused on solving this problem and a reliable therapeutic strategy is herein shown to improve sensitivity and reduce resistance to PARPi in *BRCA1*/*2* mutated tumours.

### DESCRIPTION OF THE INVENTION

### Brief description of the invention

The invention is defined by the appended claims.

As explained above, the present invention provides a reliable therapeutic strategy for improving sensitivity and reducing resistance to PARPi in *BRCA1*/*2* mutated tumours. This reliable therapeutic strategy comprises the use of Cx43 in the treatment of *BRCA1*/*2* mutated cancer, preferably selected from: *BRCA1*/*2* mutated triple negative breast cancer (TNBC) or *BRCA1*/*2* mutated ovarian cancer or *BRCA1*/*2* mutated lung cancer. In a preferred embodiment, Cx43 is administered in combination with the PARPi.

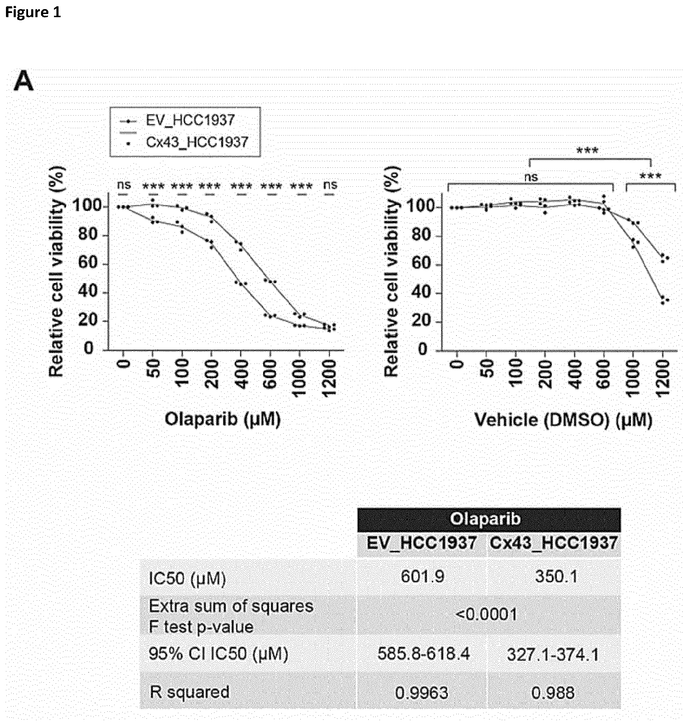

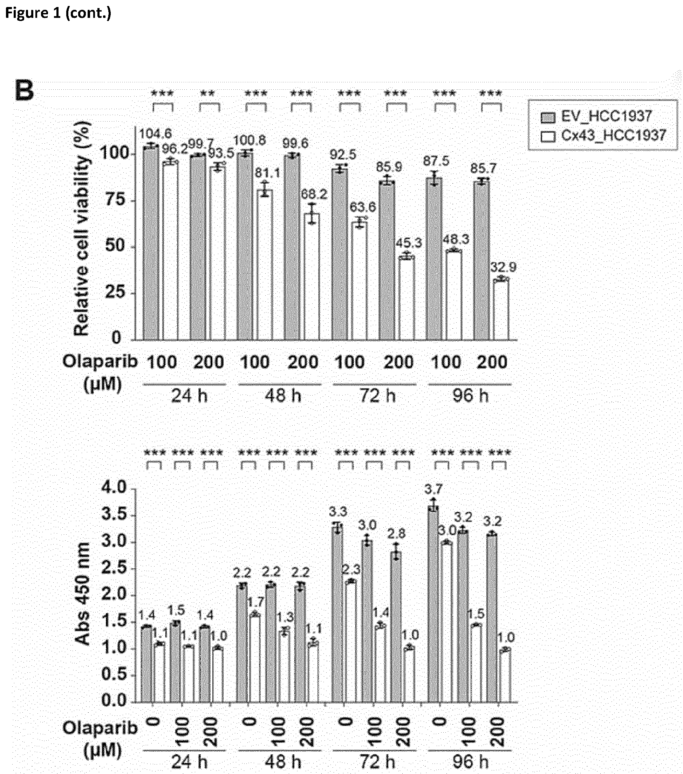

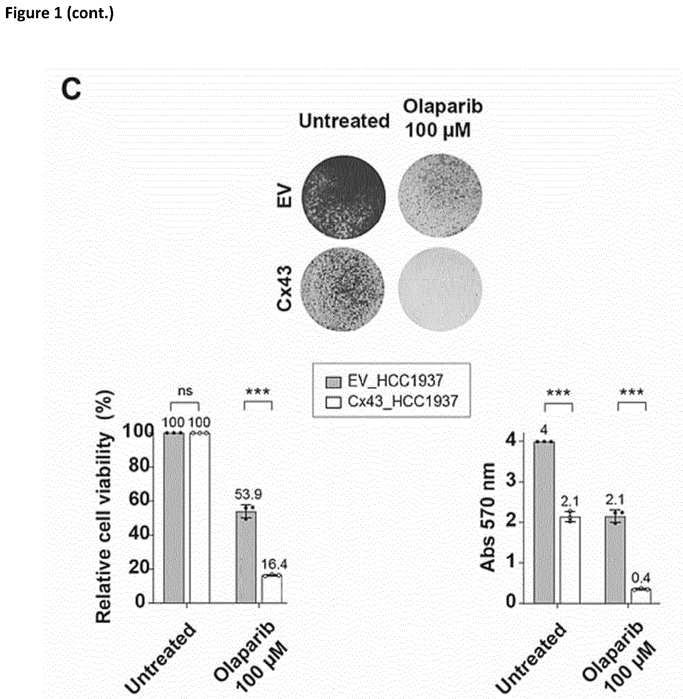

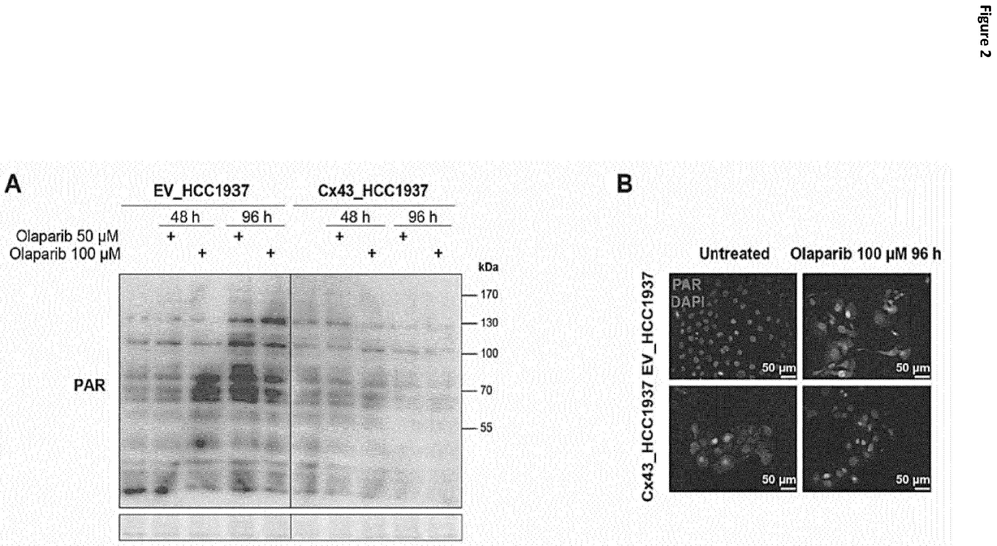

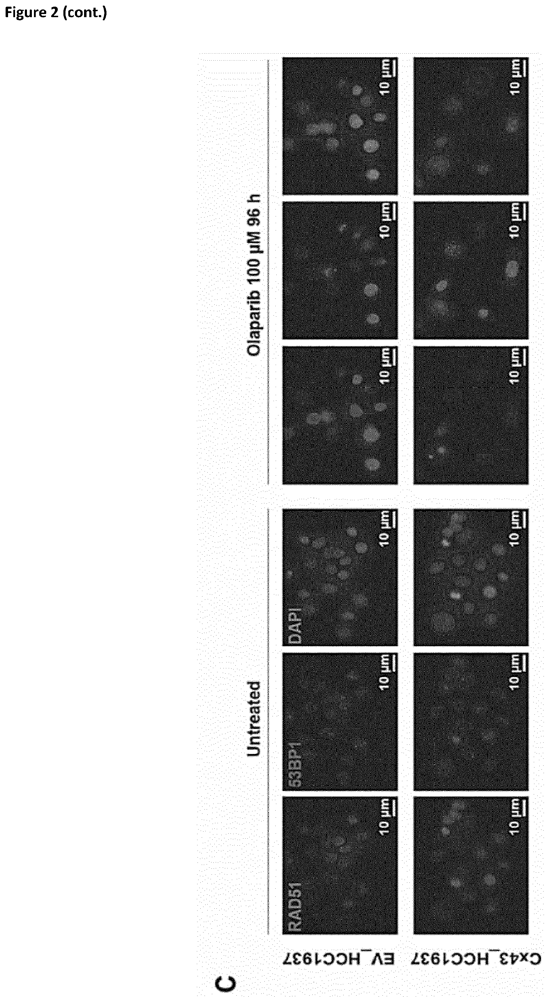

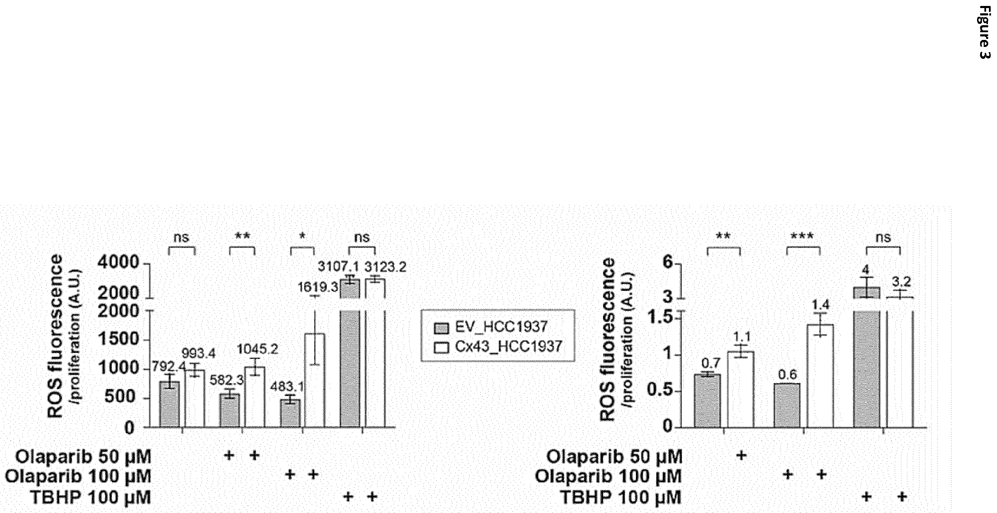

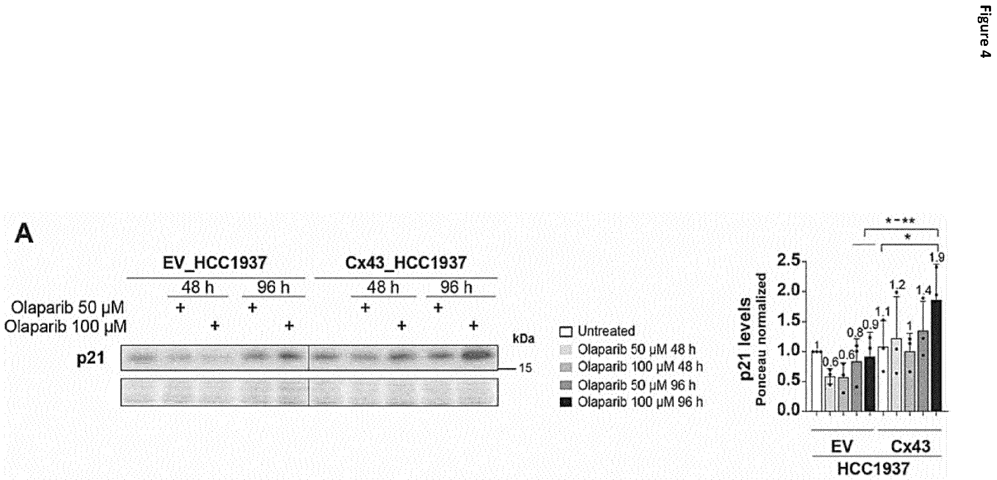

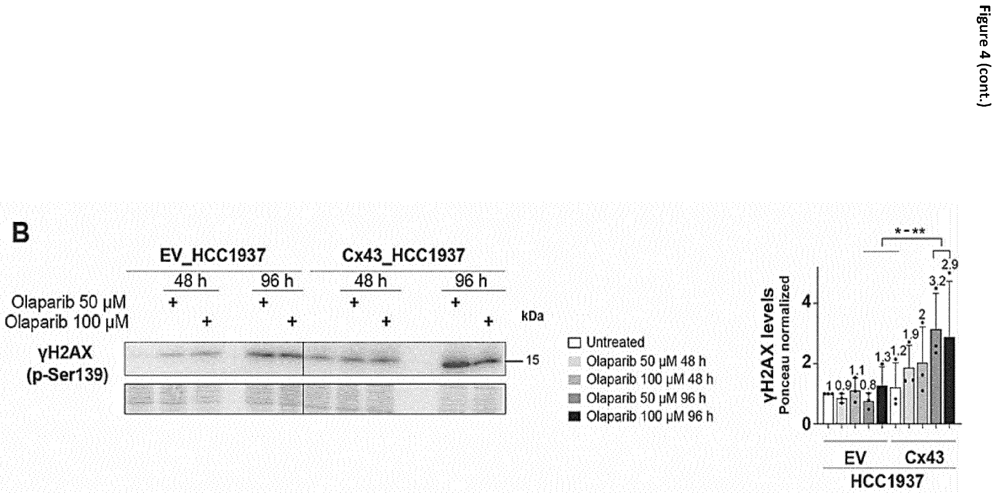

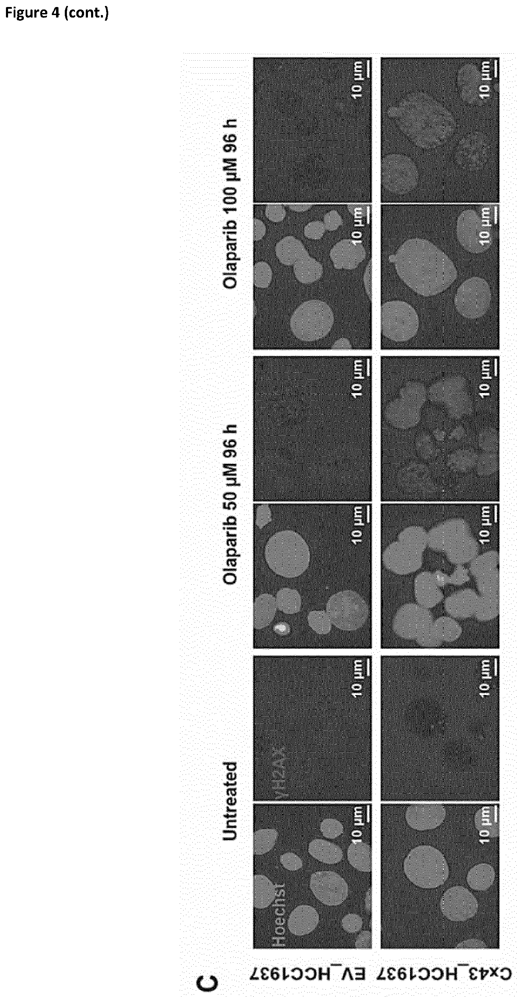

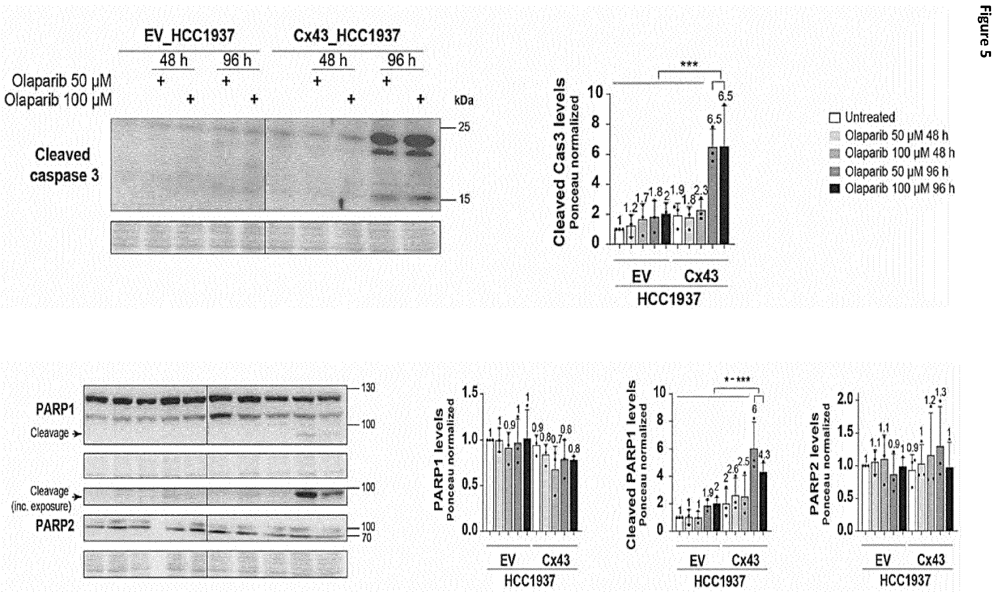

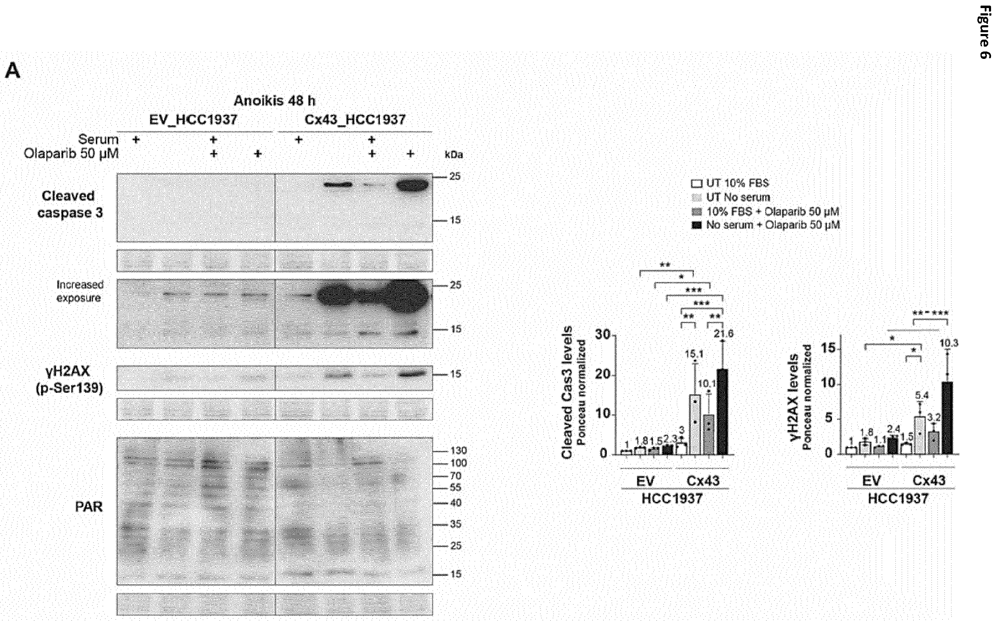

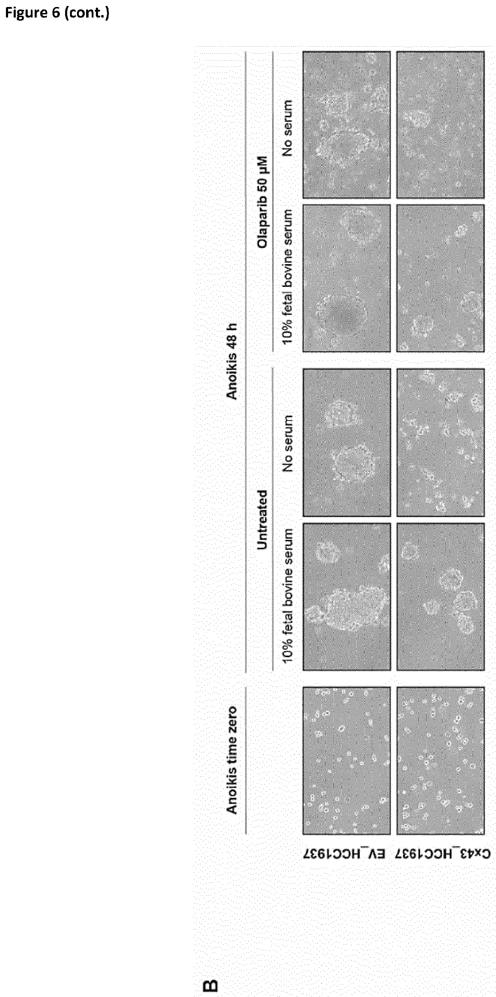

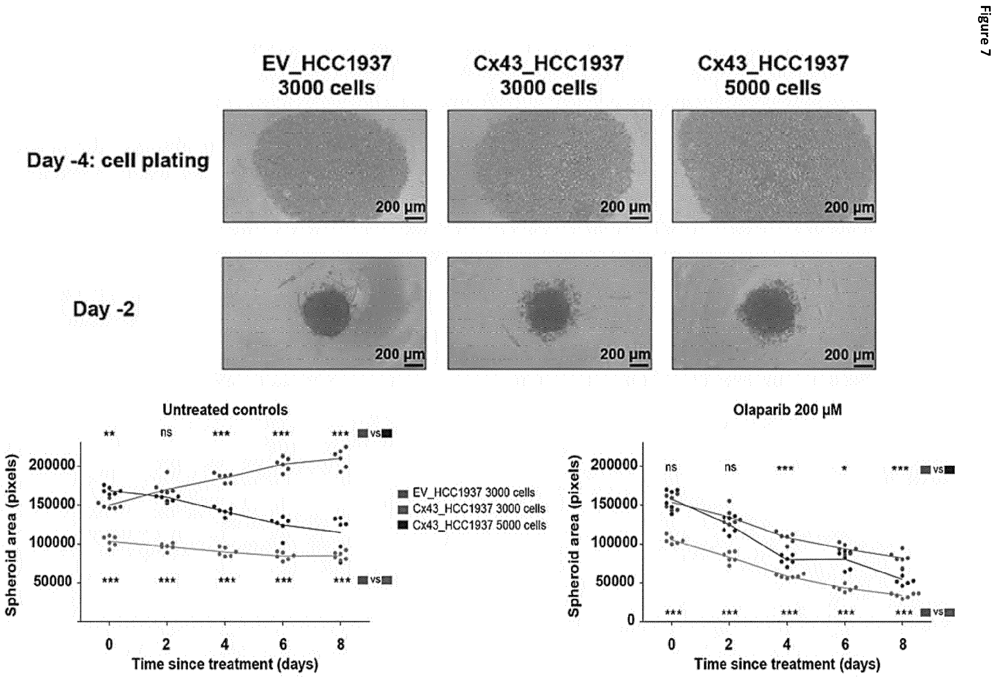

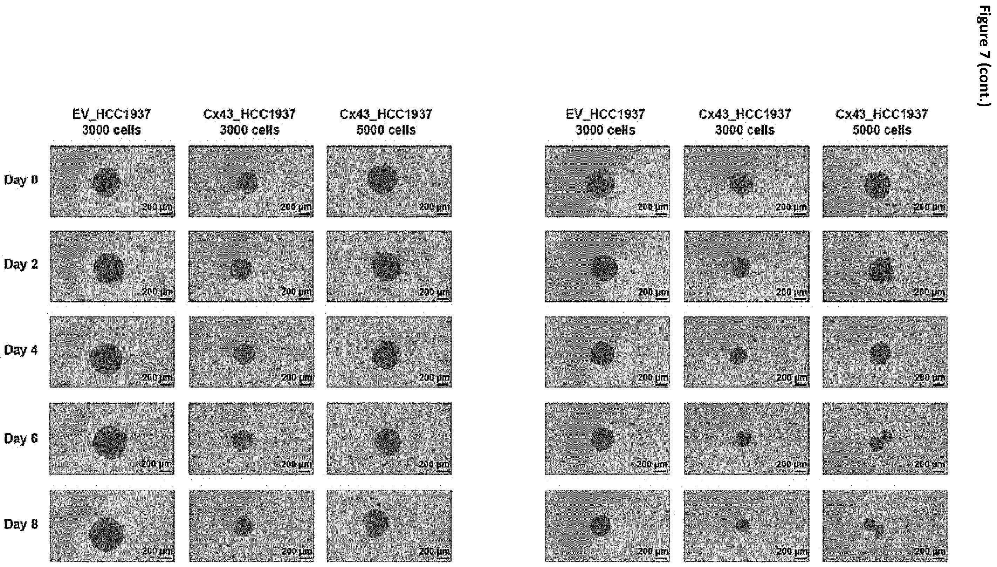

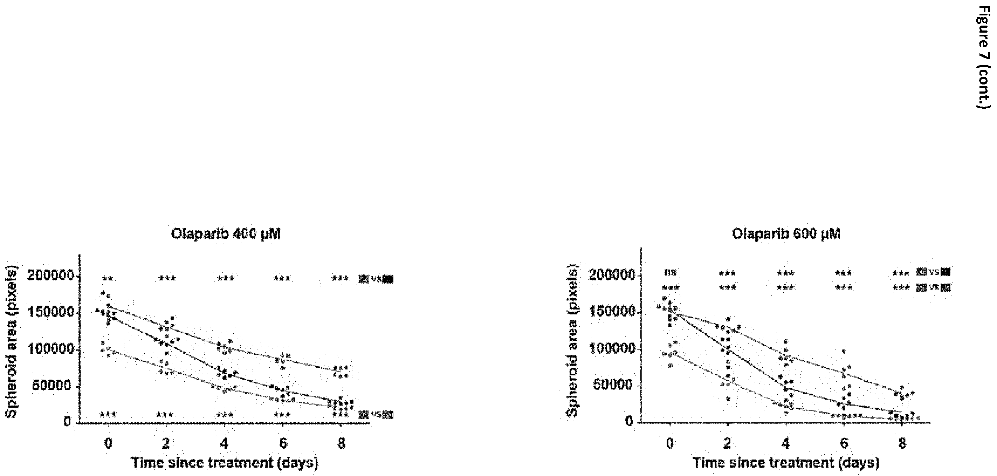

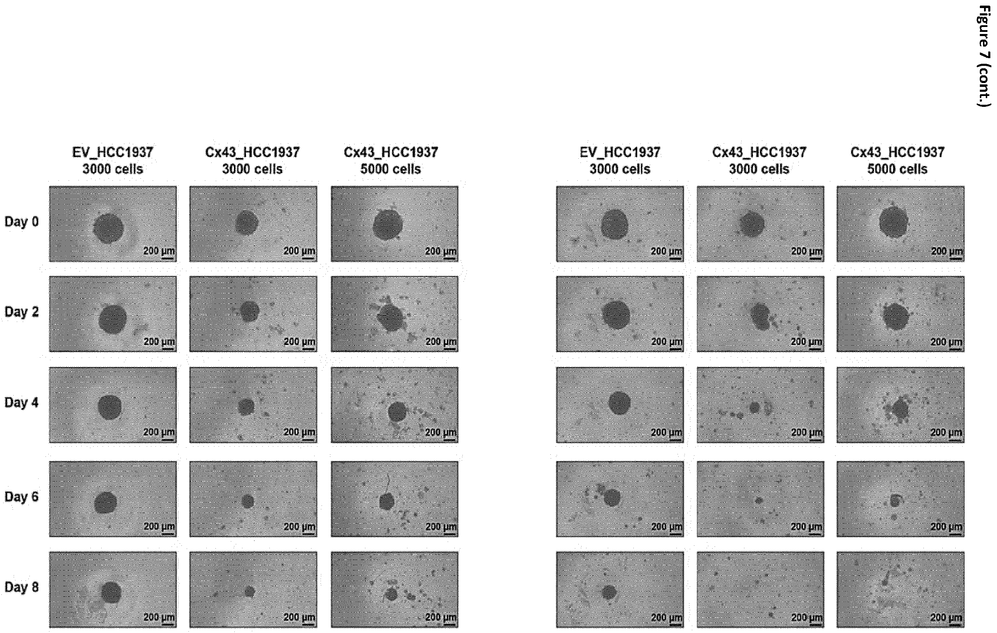

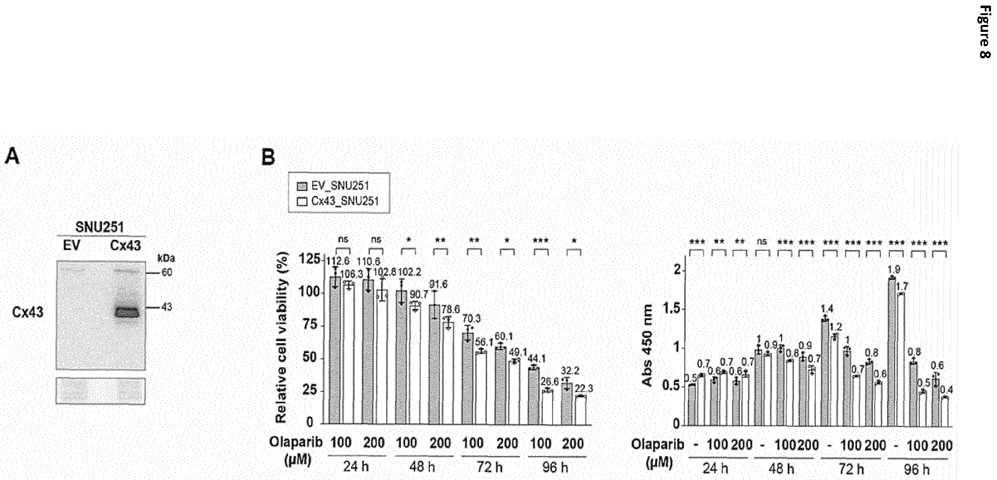

Particularly, the results provided by the present invention offer novel strategies to overcome *de novo* PARPi (olaparib) resistance in triple negative *BRCA1*-mutated breast cancer (**Figure 1** to **Figure 7**) and to enhance olaparib sensitivity in *BRCA1*-mutated ovarian cancer (**Figure 8**). Cx43 restoration in these *de novo* resistant cancer cells leads to a significant resensitization to olaparib treatment, reducing their IC50 almost by half in comparison with wild type cells (**Figure 1****.A**). This approach steeply reduces tumoral proliferative and colony forming abilities in 2D culture (**Figure 1****.B-C**) and 3D spheroids (**Figure 7**) and increases dsDNA damage accumulation (**Figure 4****.B-C**) and reactive oxygen species (ROS) production (**Figure 3**). It also diminishes PARP enzymes response by decreasing total protein PARylation (**Figure 2****.A-B**), eventually leading to an acute increment in programmed cell death by apoptosis, both in normal culture (**Figure 5****.A**) and suspension-forced/Anoikis-inducing conditions (**Figure 6****.A**).

The conclusions drawn from this work may benefit *BRCA1*/*2*-mutated olaparib-amenable cancer types, which include breast, pancreatic, ovarian, lung and prostate carcinomas, thus heavily increasing the impact of this novel therapeutic strategy within the promising field of PARPi.

So, the first embodiment of the present invention refers to Cx43 for use in the treatment of *BRCA1*/*2* mutated cancer selected from: *BRCA1*/*2* mutated triple negative breast cancer (TNBC), *BRCA1*/*2* mutated ovarian cancer and/or *BRCA1*/*2* mutated lung cancer; wherein the delivery vehicle is a nanoparticle, an extracellular vesicle or an expression vector which encodes Connexin 43.

In a preferred embodiment, Cx43 is administered before, after or simultaneously (i.e., concomitantly) to a treatment with a PARPi.

In a preferred embodiment, Cx43 is administered before, after or simultaneously (i.e., concomitantly) to a treatment with a PARPi selected from: Olaparib, niraparib, veliparib, talazoparib or rucaparib.

Alternatively, the present invention refers to a method for treating patients suffering from *BRCA1*/*2* mutated cancer selected from: *BRCA1*/*2* mutated triple negative breast cancer (TNBC) or *BRCA1*/*2* mutated ovarian cancer or *BRCA1*/*2* mutated lung cancer, which comprises the administration of a therapeutically effective dose or amount of Cx43, preferably in combination with a PARPi, or a pharmaceutical composition comprising thereof; wherein the delivery vehicle is a nanoparticle, an extracellular vesicle or an expression vector which encodes Connexin 43.

The second embodiment of the present invention refers to a combination drug product (hereinafter *"combination drug product of the invention*") comprising Connexin 43 and a PARPi; wherein the delivery vehicle is a nanoparticle, an extracellular vesicle or an expression vector which encodes Connexin 43.

In a preferred embodiment, the combination drug product comprises Cx43 selected between Cx43 protein, mRNA or DNA.

In a preferred embodiment, the combination drug product comprises Cx43 and a PARPi selected from: Olaparib, niraparib, veliparib, talazoparib or rucaparib.

The third embodiment of the present invention refers to a pharmaceutical composition (hereinafter *"pharmaceutical composition of the invention*") comprising the combination drug product of the invention and, optionally, pharmaceutically acceptable excipients or carriers; wherein the delivery vehicle is a nanoparticle, an extracellular vesicle or an expression vector which encodes Connexin 43.

In a preferred embodiment, the present invention refers to the pharmaceutical composition of the invention for use in the treatment of *BRCA1*/*2* mutated cancer, preferably selected from: *BRCA1*/*2* mutated TNBC, *BRCA1*/*2* mutated ovarian cancer and/or *BRCA1*/*2* mutated lung cancer.

In a preferred embodiment, the delivery vehicle, to administer Cx43, is a nanoparticle, an extracellular vesicle or an expression vector which encodes Cx43. Particularly, any of the extracellular vesicles known in the prior art may be used for this purpose [Guillaume van Niel, et al., 2018. Shedding light on the cell biology of extracellular vesicles. Nature Reviews Molecular Cell Biology volume 19, pages 213-228(2018*)*] [Oscar P. B. Wiklander et al., 2019. Advances in therapeutic applications of extracellular vesicles. Science Translational Medicine. 15 May 2019. Vol. 11, Issue 492, eaav8521. DOI: 10.1126/scitranslmed.aav8521].

In the context of the present invention the following terms are defined:
- The term "comprising" means including, but it is not limited to, whatever follows the word "comprising". Thus, use of the term "comprising" indicates that the listed elements are required or mandatory, but that other elements are optional and may or may not be present.
- By "consisting of" means including, and it is limited to, whatever follows the phrase "consisting of". Thus, the phrase "consisting of" indicates that the listed elements are required or mandatory, and that no other elements may be present.
- "Pharmaceutically acceptable excipient or carrier" refers to an excipient that may optionally be included with the pharmaceutical composition of the invention and that causes no significant adverse toxicological effects to the patient.
- By "therapeutically effective dose or amount" of the pharmaceutical composition of the invention is intended an amount that, when administered as described herein, brings about a positive therapeutic response in a subject having cancer. The exact amount required will vary from subject to subject, depending on the age, and general condition of the subject, the severity of the condition being treated, mode of administration, and the like. An appropriate "effective" amount in any individual case may be determined by one of ordinary skill in the art using routine experimentation, based upon the information provided herein.

### Description of the figures

**Figure 1****. Cx43 significantly resensitizes *BRCA1* mutated triple negative breast cancer cells to the PARP inhibitor olaparib. A.** 48 h dose-response curve of control empty vector (EV, red) and Cx43-restituted (green) HCC1937 cells treated with various concentrations of PARPi olaparib (0-1200 µM). Cell viability for each treatment was normalized against the cell viability of their respective untreated controls. It is included the dose-response curve of olaparib vehicle, dimethyl sulfoxide (DMSO), to account for potential DMSO cytotoxicity at higher drug concentrations. n=3, each n is represented as a dot in the graph. T-test corrected for multiple comparisons using the Holm-Šídák method. *P<0.05, **P<0.01, ***P<0.001, ns=not significant. It is also specified the dose-response curve analysis parameters, including the half maximal inhibitory concentration IC50 (µM), extra sum of squares F test p-value, 95% confidence interval (CI) of the IC50 (µM) and R squared. Extra sum-of-squares F test was performed to evaluate differences in best-fit parameter logIC50 among cell lines. **B.** Proliferation assay of EV (grey) and Cx43_HCC1937 (white) cells over the course of 96 h, taking measurements every 24 h, under 100 and 200 µM olaparib treatment. Proliferation values were either normalized against their respective untreated controls for each time point (top graph), or raw absorbance proliferation data were reported (bottom graph). n=3, each n is represented as a dot in the graph, mean+SD, mean values specified in the graphs. T-test corrected for multiple comparisons using the Holm-Šídák method. *P<0.05, **P<0.01, ***P<0.001, ns=not significant. **C.** Colony formation assay of EV (grey) and Cx43_HCC1937 (white) cells treated with 100 µM olaparib over the course of 7 days. Cell viability for each treatment was normalized against the cell viability of their respective untreated controls (left graph) or expressed as raw absorbance values (right graph). N=3, each n is represented as a dot in the graph; mean+SD; mean values specified in the graph. T-test. *P<0.05, **P<0.01, ***P<0.001, ns=not significant.
**Figure 2****. Cx43 reduces global protein PARylation and is associated with lower levels of RAD51 in *BRCA1* mutated triple negative breast cancer cells under olaparib treatment. A-B**. Cx43 restitution in HCC1937 cells reduces global protein PARylation during olaparib treatment. Western blot (**A**) and immunofluorescence (**B**) against poly (ADP-ribose) (PAR) polymer (red) in 50/100 µM olaparib-treated EV and Cx43_HCC1937 cells for 48 and 96 h. Ponceau S Acid Red is shown as total protein loading control (A). Nuclei are stained with DAPI (blue, B). **C.** EV_HCC1937 cells have higher RAD51 in response to olaparib than Cx43_HCC1937 cells. Representative immunofluorescence against RAD51 (red) and 53BP1 (green) in EV and Cx43_HCC1937 cells treated with 100 µM olaparib for 96 h. Nuclei are stained with DAPI (blue).
**Figure 3****. Cx43 significantly increases intracellular reactive oxygen species (ROS) production in *BRCA1* mutated triple negative breast cancer cells during olaparib treatment.** Fluorescence-based quantitation of intracellular reactive oxygen species (ROS) production in EV (grey) and Cx43_HCC1937 cells (white) treated with 50/100 µM olaparib for 96 h. 100 µM TBHP treatment was included as a positive control of ROS production. All measurements were relativized to cellular density in each of the conditions (left graph); each experimental condition was further normalized against its untreated counterpart (right graph). Mean+SD; mean values specified in the graph. n=3, each n is represented as a dot in the graph. T-test. *P<0.05, **P<0.01, ***P<0.001, ns=not significant.
**Figure 4****. Olaparib significantly increases p21-mediated senescence and DNA damage accumulation in Cx43-restituted *BRCA1* mutated triple negative breast cancer cells. A.** Cx43 restitution in HCC1937 cells leads to p21-mediated senescence after olaparib treatment. Western blot against senescence marker p21 in 50/100 µM olaparib-treated EV and Cx43_HCC1937 cells for 48 and 96 h. Ponceau S Acid Red is shown as total protein loading control. Graphs represent protein expression for all conditions relativized to their respective Ponceau loading values, and then normalized against the expression of untreated EV_HCC1937 cells. Mean+SD; mean values specified in the graph. N=3, each n is represented as a dot in the graph. One-way ANOVA with Fisher's Least Significant Difference (LSD) test for specific and relevant comparisons. *P<0.05, **P<0.01, ***P<0.001. **B.** Cx43 restitution in HCC1937 cells results in DNA damage and senescence marker γH2AX (p-Ser139) accumulation after olaparib treatment. Western blot against γH2AX (p-Ser139) was performed after 48 and 96 h of 50/100 µM olaparib treatment in EV and Cx43_HCC1937 cells. Ponceau S Acid Red is shown as total protein loading control. Graphs represent protein expression for all conditions relativized to their respective Ponceau loading values, and then normalized against the expression of EV _HCC1937 untreated cells. Mean+SD; mean values specified in the graph. n=3, each n is represented as a dot in the graph. One-way ANOVA with Fisher's Least Significant Difference (LSD) test for specific and relevant comparisons. *P<0.05, **P<0.01, ***P<0.001. **C.** Representative immunofluorescence against γH2AX (green) in 50 and 100 µM olaparib-treated EV and Cx43_HCC1937 cells for 96 h. Nuclei are stained with Hoechst (blue). n=2.
**Figure 5****. Cx43 significantly increases programmed cell death in *BRCA1* mutated triple negative breast cancer cells under olaparib treatment.** Cx43 restitution in HCC1937 cells enhances apoptosis by caspase 3 and PARP-1 cleavage after olaparib treatment. Western blot against cleaved caspase 3, PARP-1 and PARP-2 was performed after 48 and 96 h of 50/100 µM olaparib treatment in EV and Cx43_HCC1937 cells. Ponceau S Acid Red is shown as total protein loading control. Graphs represent protein expression for all conditions relativized to their respective Ponceau loading values, and then normalized against the expression of EV _HCC1937 untreated cells. Mean+SD; mean values specified in the graph. n=3 (n=1 for caspase 7), each n is represented as a dot in the graph. One-way ANOVA with Fisher's Least Significant Difference (LSD) test for specific and relevant comparisons. *P<0.05, **P<0.01, ***P<0.001.
**Figure 6****. Cx43 significantly increases programmed cell death and DNA damage and reduces global PARylation in *BRCA1* mutated triple negative breast cancer cells during olaparib treatment under Anoikis-inducing conditions. A.** Western blot against cleaved caspase 3, γH2AX (p-Ser139) and PAR in 50 µM olaparib-treated EV and Cx43_HCC1937 cells cultured in the presence and absence of fetal bovine serum (FBS) 10% under Anoikis-inducing conditions for 48 h. Ponceau S Acid Red is shown as total protein loading control. 20 kDa-Cx43 isoform is highlighted by a dashed rectangle in the blot. Graphs represent protein expression for all conditions relativized to their respective Ponceau loading values, and then normalized against the expression of untreated (UT) EV_HCC1937 or untreated Cx43_HCC1937 cells (20 kDa-Cx43 and vimentin quantitations). Mean+SD; mean values specified in the graph. n=3, each n is represented as a dot in the graph. One-way ANOVA with Fisher's Least Significant Difference (LSD) test for specific and relevant comparisons. *P<0.05, **P<0.01, ***P<0.001. **B.** Representative images of EV and Cx43_HCC1937 cells cultured in forced-suspension Anoikis-inducing conditions at times 0 and 48 h, in the presence/absence of 10% FBS and 50 µM olaparib.
**Figure 7****. Olaparib treatment in Cx43-restituted *BRCA1* mutated triple negative breast cancer cells leads to significantly lower 3D spheroid proliferation than in wild type cells.** Representative phase contrast images of EV and Cx43_HCC1937 spheroids plated at 3000 and 3000-5000 initial cell densities, respectively, under ultra-low attachment conditions (day -4) and, once they were 4 days old (day 0), treated with olaparib 200, 400 and 600 µM for a total of 8 days (days 0-8). Their size was individually tracked and photographed every 48 h. EV spheroids are shown in red and Cx43 spheroids in green (and also in blue for Cx43_HCC1937 plated at 5000 initial cell density). Graphs represent spheroid area in pixels. n=5-6, each n is represented as a dot in the graph. T-test. *P<0.05, **P<0.01, ***P<0.001, ns=not significant.
**Figure 8****. Cx43 restitution significantly resensitizes *BRCA1* mutated ovarian cancer cell line SNU251 to olaparib treatment. A.** Representative western blot against Cx43 in EV and Cx43_SNU251 cells. Ponceau S Acid Red is shown as total protein loading control. **B.** Proliferation assay of EV (grey) and Cx43-restituted (white) SNU251 cells treated with 100/200 µM olaparib for 96 h. Proliferation values were either normalized against their respective untreated controls for each time point (left graph), or raw absorbance proliferation data were reported (right graph). n=3, each n is represented as a dot in the graphs, mean+SD, mean values specified in the graphs. T-test corrected for multiple comparisons using the Holm-Šídák method. *P<0.05, **P<0.01, ***P<0.001, ns=not significant.

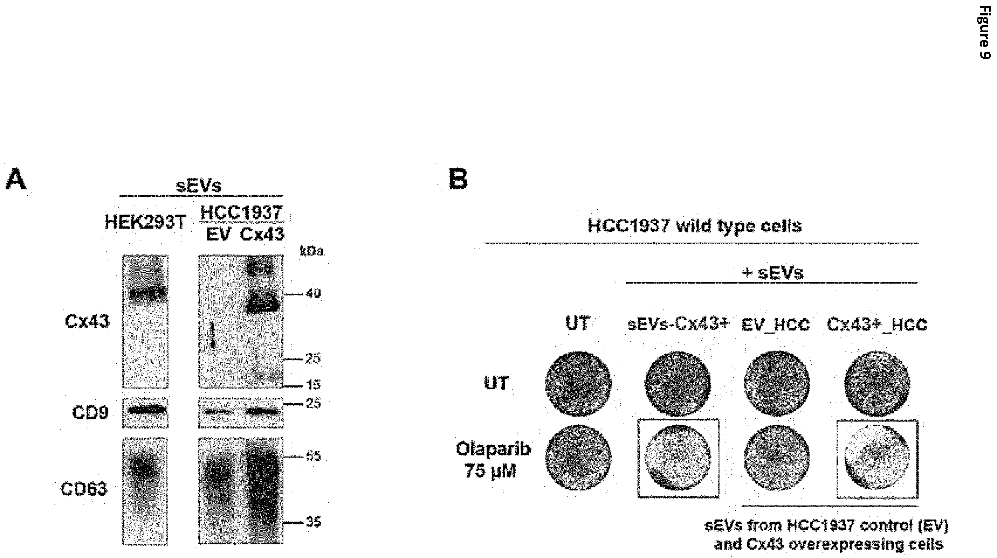

**Figure 9****. Small extracellular vesicles (sEVs) containing Cx43 improve olaparib efficacy in *de novo* resistant *BRCA1* mutated HCC1937 cells. A**. Representative western blot against Cx43 in HEK-293T and EV/Cx43_HCC1937-derived sEVs. The tetraspanin proteins CD9 and CD63 are included as sEV markers. **B.** Colony formation assay of HCC1937 wild type cells treated with olaparib 75 µM, Cx43-null (EV_HCC sEVs) or Cx43-positive sEVs (HEK-293T-sEVS, named sEVs-Cx43+ in the figure, and Cx43_HCC1937-sEVs, named Cx43+_HCC, highlighted with red squares) and their combinations for 5 days. Raw absorbance proliferation data are reported. n=1 (2 replicates), mean values specified in the graph.

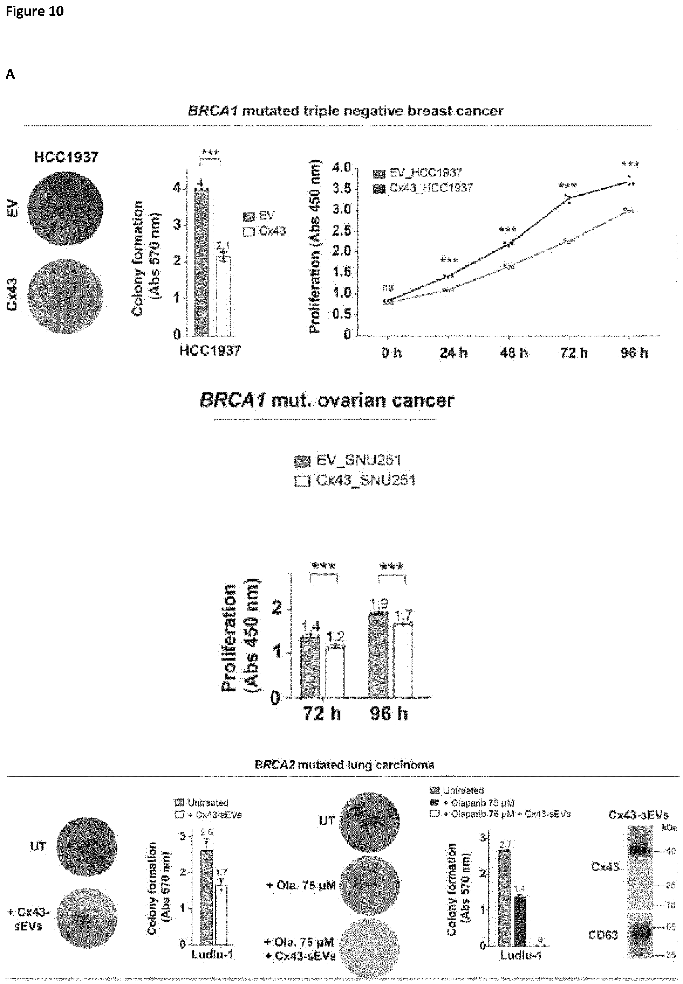

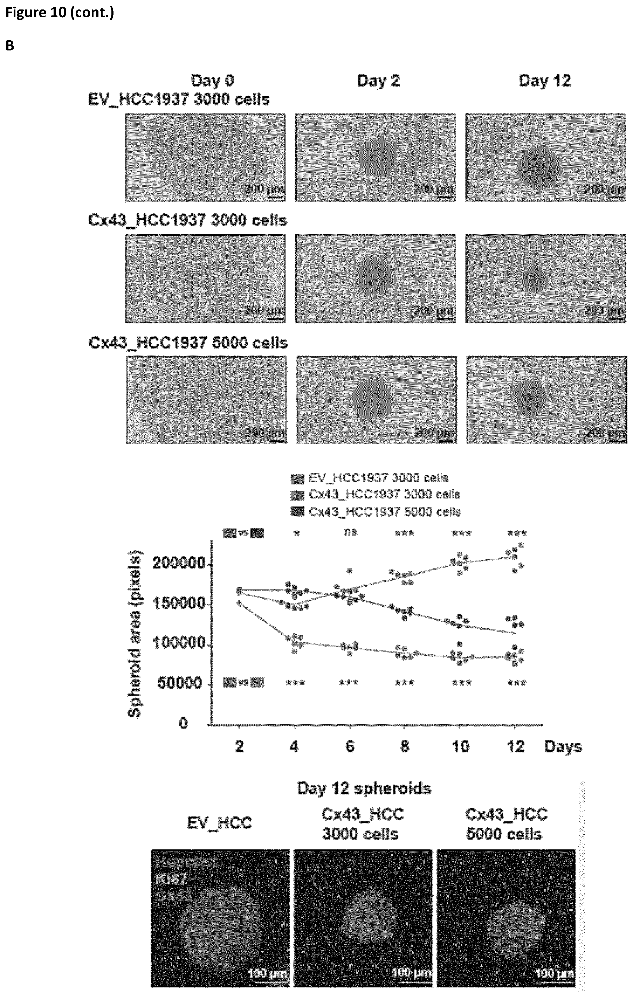

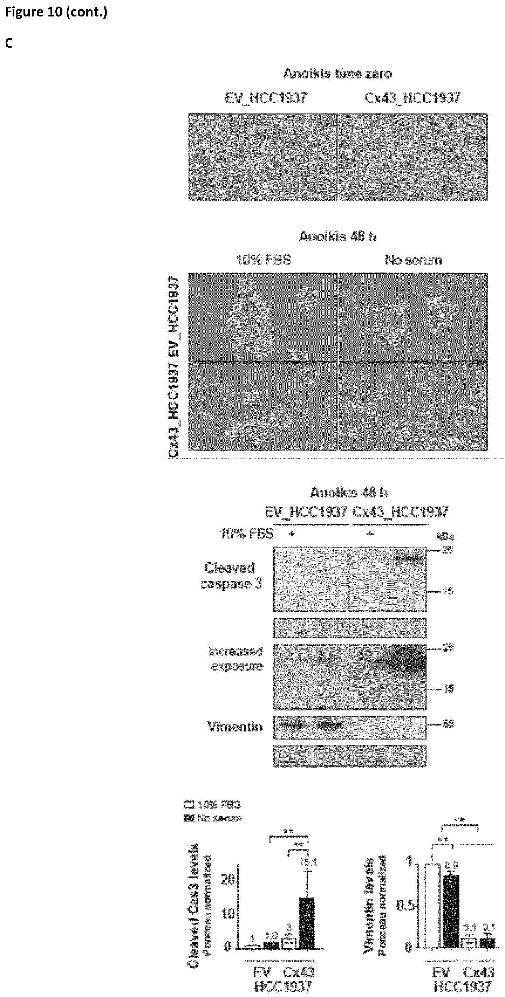

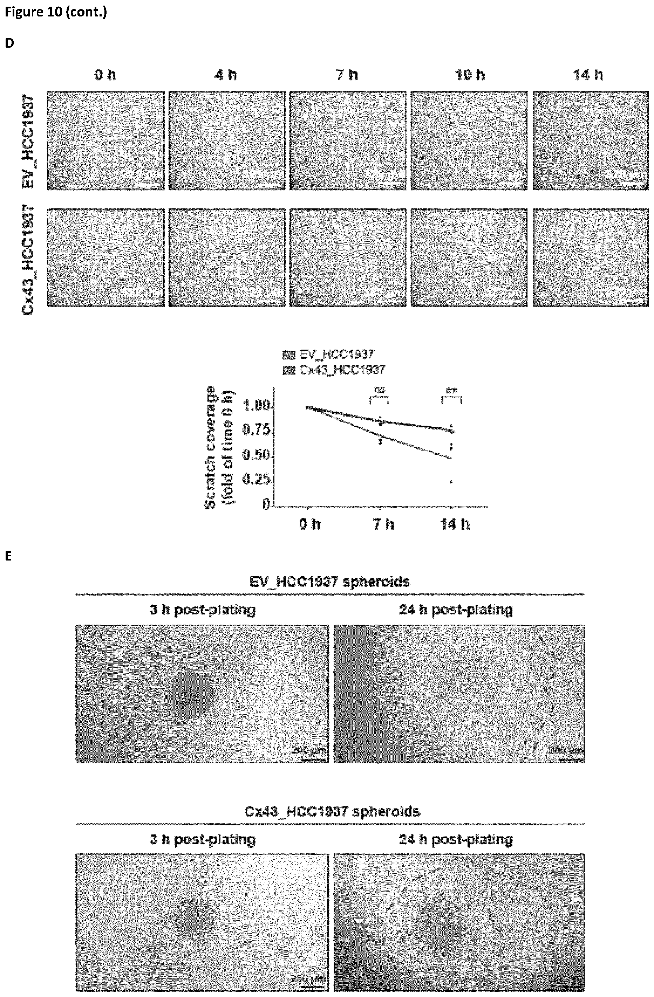

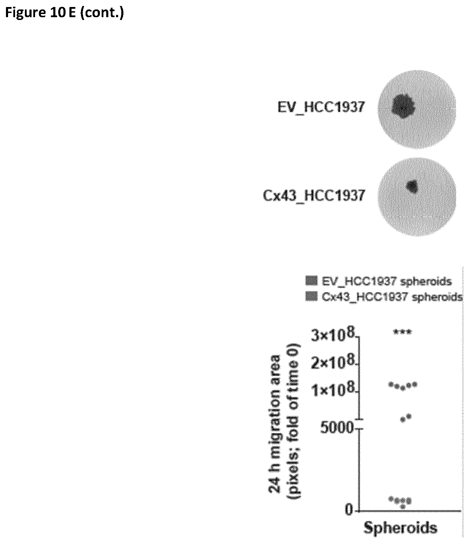

**Figure 10****. Role of Cx43 as a tumour suppressor in *BRCA1*/*2* mutated cancer. A. Cx43 restoration significantly reduces proliferation and colony formation in *BRCA* mutated triple negative breast cancer (TNBC), ovarian and lung carcinoma cell lines. Small extracellular vesicles (sEVs) containing Cx43 improve olaparib efficacy in *BRCA2* deficient lung cancer Ludlu-1 cells.** (Left) Colony formation assay of EV and Cx43-restored *BRCA1* mutated TNBC HCC1937 cells over the course of 6 days. EV_HCC1937 cells are shown in grey and Cx43-restituted in white. Mean+SD; mean values specified in the graph n=3, each n is represented as a dot in the graph. T-test. *P<0.05, **P<0.01, ***P<0.001. (Center) Proliferation assay of EV and Cx43-restored HCC1937 and *BRCA1* mutated ovarian carcinoma SNU251 cells over the course of 96 h, taking measurements every 24 h. EV cells are shown in black/grey and Cx43-restituted in green/white. n=3, each n is represented as a dot in the graph. T-test corrected for multiple comparisons using the Holm- Šídák method. *P<0.05, **P<0.01, ***P<0.001. (Right) Colony formation assay of *BRCA2* mutated lung cancer Ludlu-1 wild type cells treated for 5 days with Cx43-positive sEVs (Cx43-sEVs), derived from non-tumoral HEK293T cells, olaparib 75 µM and their combination. Raw absorbance proliferation data are reported. 2 replicates, mean values specified in the graph, each replicate is represented as a dot in the graph. A representative western blot can be seen on the right against Cx43-sEVs isolated from the non-tumoral cells enriched in Cx43. The typical sEVs marker tetraspanin protein CD63 is included as a control. **B. Cx43 restitution significantly reduces 3D proliferation in *BRCA1* mutated TNBC cells.** Representative pics of EV and Cx43_HCC1937-derived spheroids along time. HCC1937 spheroids were plated at an initial density of 3000 (EV and Cx43) and 5000 (Cx43) cells and analysed every 2 days for a total period of 12 days. EV spheroids are shown in red and Cx43 spheroids in green (and also in blue for Cx43_HCC1937 plated at 5000 cells initial density). Graphs represent spheroid area in pixels. n=6, each n is represented as a dot in the graph. T-test. *P<0.05, **P<0.01, ***P<0.001. Representative confocal immunofluorescences (maximum intensity projection) against Ki67 (green) and Cx43 (red) in 12-day old EV (3000 initial cell density) and Cx43_HCC1937 (3000 and 5000 initial cell density) spheroids. Nuclei were stained with Hoechst (blue). Z-stacks were acquired with a step size of 15 µm. **C. Cx43 restoration significantly resensitizes *BRCA1* mutated TNBC cells to Anoikis.** Representative phase contrast images at 0 h and 48 h of EV and Cx43_HCC1937 cells grown under suspension-forced Anoikis-inducing conditions, in the presence (white) and absence (black) of fetal bovine serum (FBS) 10%. Cell aggregates are indicated by dashed red lines. Western blot against cleaved caspase 3 and vimentin was performed after 48 h Anoikis induction. Ponceau S Acid Red is shown as total protein loading control. Graphs represent cleaved caspase 3 and vimentin expression for all conditions relativized to their respective Ponceau loading values, and then normalized against EV _HCC1937 cells grown in 10% FBS. Mean+SD; mean values specified in the graph. n=3, each n is represented as a dot in the graph. One-way ANOVA with Fisher's Least Significant Difference (LSD) test for specific and relevant comparisons. *P<0.05, **P<0.01, ***P<0.001. **D-E. Cx43 restoration significantly reduces 2D (D) and 3D-spheroid (E) migration in *BRCA1* mutated TNBC cells. D.** Representative time-lapse pics of scratch/wound healing assay performed in EV and Cx43 HCC1937 cells for 14 h. EV cells are shown in black and Cx43-restituted in green. Graph represents the area of scratch coverage at 0, 7 and 14 h, that is, the surface that has not been covered by migrating cells yet, which is represented as a fold of the scratch coverage at time 0 h (set to 1). n=3, each n is represented as a dot in the graph. T-test. *P<0.05, **P<0.01, ***P<0.001. **E.** Representative phase contrast images of 3-day-old EV (red) and Cx43(green)_HCC1937 spheroids 3 hours post-plating in gelatin coated plates for migration assay, and their 24 h post-plating follow up. The migration front is indicated by dashed red lines. 24 h post-plating spheroids are also shown stained with crystal violet. Graph represents the migrated area of each spheroid at 24 h quantitated in pixels, normalized to the respective initial spheroid area at 3 h post-plating. n=7, each n is represented as a dot in the graph. T-test. *P<0.05, **P<0.01, ***P<0.001.

### Detailed description of the invention

The present invention is illustrated by means of the Examples set below without the intention of limiting its scope of protection.

### Example 1. Material and Methods

### Example 1.1. Cell lines and culture conditions

Established human triple negative breast cancer (HCC1937), ovarian carcinoma (SNU251), lung cancer (Ludlu-1) and embryonic kidney (HEK293T) cell lines were used in the development of this patent. Their main characteristics and culture conditions are summarized in **Table 1.** All cells were cultured in a humidified incubator (SANYO CO2) at 37 °C and 5 % CO₂, with medium change every second or third day. All cells used in experiments were low to middle passage, generally within the first 10-15 passages after thawing the cell stocks.

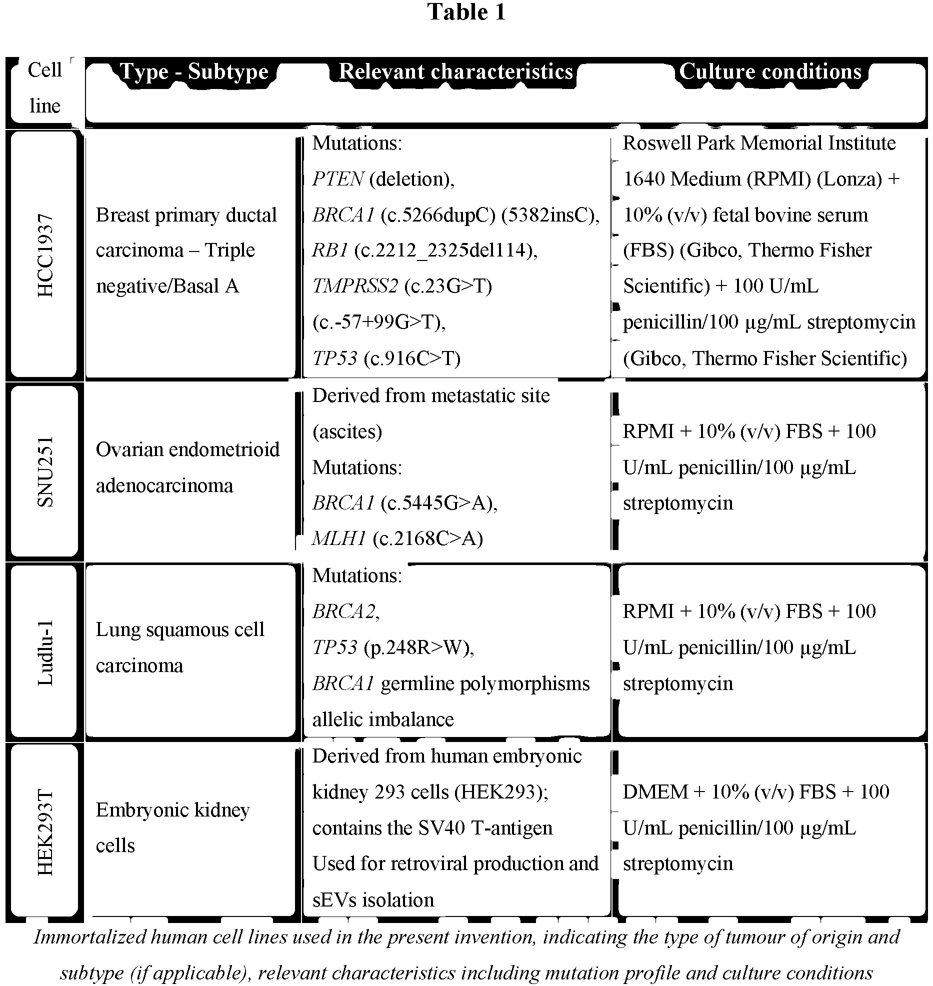

Cell lines were authenticated by short tandem repeat genotyping conducted by the Genomics Core Facility at Instituto de Investigaciones Biomédicas Alberto Sols (IIBM), Madrid. All cell lines used in the experiments were Mycoplasma-free, and Mycoplasma testing was routinely performed.

### Example 1.2. Bacteria transformation and cell lines plasmid transfection/infection procedures

### Bacteria transformation

1 µL of plasmid DNA (stock 700-1000 ng/µL) was added to 20 µL of DH5α *E.coli* competent cells and left on ice for 30 min. Then, bacteria were heat shocked at 42 °C for 30 s and left on ice for 5 min. 100 µL of LB Broth (Miller) (Sigma-Aldrich) were added to the mix and incubated in a shaker at 37 °C 1 h. Transformed bacteria were plated on LB Broth agar (Lennox) (Sigma-Aldrich) plates with 100 µg/mL ampicillin and left to grow overnight at 37 °C. The next day, single colonies were picked and cultured in LB Broth (Miller) with 100 µg/mL ampicillin overnight at 37 °C. Negative controls of bacterial transformation and expansion were run in parallel.

Plasmid DNA comprise a pIRESpuro2 vector (Clontech) with the human Cx43 sequence, along with its empty vector (EV) counterpart.

### Plasmid DNA isolation

Plasmid DNA was isolated from the previously transformed bacteria withe the GeneJET Plasmid Miniprep Kit (Thermo Fisher Scientific), following manufacturer's instructions, and DNA concentration was determined with a Nanodrop ND-1000.

### Cell line plasmid transfection

TNBC cell line HCC1937 and ovarian carcinoma SNU251 cells were transfected by electroporation with the pIRESpuro2_EV/Cx43 plasmids using the Amaxa Cell Line Nucleofector Kit V (Lonza) in the Cell Line Nucleofector Device (Lonza), according to manufacturer's instructions. Briefly, 1 million 80 % confluent pelleted cells were carefully resuspended first in 100 µL Cell Line Nucleofector Solution and then 3 µg plasmid DNA were added. Cells were then transferred to a cuvette and electroporated in the U-028 program. Transfected and mock-transfected cells were then plated in their respective growth medium without antibiotics and incubated overnight. The next day, puromycin (Tocris Bioscience) selection was initiated in complete growth medium, and antibiotic concentration was gradually increased until all mock-transfected cells were dead. Efficient cell transfection was later assessed by western blot against Cx43 (see **Table 3** for references).

### Example 1.3. Therapeutic treatments of cell lines

Immortalized tumour cell lines were treated with olaparib, whose main characteristics are summarized in **Table 2.** Treatments were renewed every 48 h unless specified otherwise.

**Table 2**

| Treatment | **Brand** | **Stock** |
|---|---|---|
| Olaparib/ Lynparza | Astrazeneca | 50 mM in dimethyl sulfoxide (DMSO, Sigma-Aldrich), -20 °C |

| | | |
|---|---|---|
| *Characteristics of therapeutic compound olaparib used in the present project* | | |

### Example 1.4. Immunofluorescence

### Immunofluorescence of 2D cultures

2 000-10 000 cells were plated on glass coverslips (Thermo Fisher Scientific) previously coated with poly-D-lysine hydrobromide (Sigma-Aldrich) and cultured until they were approximately 80 % confluent. They were washed with PBS, fixed with 4 % paraformaldehyde (PFA, Sigma-Aldrich) in PBS for 15 min at room temperature and rinsed twice with PBS. Next, cells were incubated with 0.1 M glycine (Sigma-Aldrich) in distilled water to quench PFA for 10 min at room temperature, and then their membranes were permeabilized with 0.2 % Triton X-100 (Sigma-Aldrich) in PBS for 10 min at room temperature. After two 5 min PBS washes, potential nonspecific antibody binding sites were blocked with 1 % bovine serum albumin (BSA; Sigma-Aldrich) diluted in PBST (PBS+0.1 % Tween 20; Sigma-Aldrich) for 30 min at room temperature. Cells were incubated overnight at 4 °C in the dark with primary antibody (see references in **Table 3**) diluted in 1 % BSA in PBST. The next day, cells were rinsed three times in PBS for 10 min each, and then incubated 1 h at room temperature in the dark with secondary antibody (see references in **Table 3**) diluted in 1 % BSA in PBST. After that, they were washed three times with PBS for 5 min each, and their nuclei were stained with 1 µg/mL Hoechst (Invitrogen, Thermo Fisher Scientific) diluted in PBS for 10 min. Finally, cells were rinsed three times with PBS for 10 min each and coverslips were mounted on glass microscope slides with 50 % (v/v) glycerol (Sigma-Aldrich) diluted in PBS, sealing their edges with nail polish and storing them at 4 °C until analysis. Alternatively, coverslips were directly mounted with ibidi mounting medium with DAPI (ibidi).

### Immunofluorescence of 3D cultures

Immunofluorescent staining of spheroids was performed following the protocol proposed by (Nürnberg et al., 2020) with subtle modifications. At least 10 spheroids/condition were harvested in microtubes, washed twice in PBS and fixed with PFA 4 % overnight at 4 °C. They were then rinsed twice with PBS and PFA was quenched by 0.5 M glycine in PBS for 1 h at 37 °C in a Thermoshake incubator shaker (Gerhardt Analytical Systems) at 95 rpm. Next, spheroids were incubated with penetration buffer (glycine 0.3 M, Triton X-100 0.2 %, DMSO 20 % in PBS) for 30 min at 37 °C and 95 rpm to enhance the later entrance of antibodies and nuclear dye. They were washed twice with PBS and blocked with blocking buffer (BSA 1 %, Triton X-100 0.2 %, DMSO 10 % in PBS) for 2 h at 37 °C and 95 rpm. Primary antibody was then diluted in antibody buffer (BSA 1 %, Tween20 0.2 %, DMSO 5 % in PBS) and incubated overnight at 37 °C and 95 rpm. The next day samples were washed 5 times with wash buffer (BSA 1 %, Tween20 0.2 % in PBS), 5 min/wash, at room temperature and gentle shaking. Secondary antibodies (see references in Table 3) and 5 µg/mL Hoechst nuclear dye were added together in antibody buffer and incubated overnight at 37 °C and 95 rpm in the dark. Spheroids were washed 5 times with wash buffer, 5 min/wash, at room temperature and gentle shaking. Optical clearing of spheroids was perfomed by incubation in 90 % (v/v) glycerol in distilled water for at least 24 h at 37 °C and 95 rpm in the dark. Samples were then mounted in 18 well 1.5H glass bottom microslides (ibidi) using 90 % (v/v) glycerol and kept at 4 °C in the dark until confocal imaging.

### Immunofluorescence imaging

Immunofluorescences of 2D cultures were photographed under an Olympus BX61 microscope coupled to a DP71 digital camera (Olympus). Confocal imaging was performed using a Nikon A1R confocal microscope (Nikon).

**Table 3**

| **List of antibodies used in this patent** | | | | |
|---|---|---|---|---|
| Target antigen-(conjugation) | Host | Applications and dilution | Manufacturer | Reference |
| 53BP1 | Rabbit | IF | Novus Biologicals | NB100-305SS |
| Cleaved caspase-3 (Asp175) | Rabbit | WB | Cell Signaling | 9661 |
| CD9 | Rabbit | WB | Santa Cruz Biotechnology | sc-9148 |
| CD63 | Rabbit | WB | Santa Cruz Biotechnology | sc-15363 |
| Connexin43 | Rabbit | WB, IF | Sigma-Aldrich | C6219 |
| Ki67 | Mouse | IF | Cell Signaling | 9449 |
| Mouse IgG-(Alexa Fluor 594) | Goat | IF | Invitrogen, Thermo Fisher Scientific | A-11032 |
| Mouse IgG-(FITC) | Goat | IF | Invitrogen, Thermo Fisher Scientific | F-2761 |
| Mouse IgG-(HRP) | Sheep | WB | Sigma-Aldrich | NA-931 |
| p21 | Rabbit | WB | Cell Signaling | 2947 |
| PAR/pADPr | Mouse | WB, IF | R&D Systems | 4335-MC-100 |
| PARP-1 | Rabbit | WB | Cusabio | CSB-PA017457LA01HU |
| PARP-2 | Rabbit | WB | FineTest | FNab06156 |
| Phospho-Histone H2A.X (Ser 139) | Mouse | WB, IF | Santa Cruz Biotechnology | sc-517348 |
| Rabbit IgG-(Alexa Fluor 568) | Goat | IF | Invitrogen, Thermo Fisher Scientific | A10042 |
| Rabbit IgG-(FITC) | Goat | IF | Invitrogen, Thermo Fisher Scientific | F-2765 |
| Rabbit IgG-(HRP) | Goat | WB | Sigma-Aldrich | A6154 |
| Rad51 | Mouse | IF | Novus Biologicals | NB100-148 |

| | | | | |
|---|---|---|---|---|
| *Antibodies used in the present project. All target antigens are human unless specified.* *Applications: western blot (WB), immunofluorescence (IF).* | | | | |

### Example 1.5. Protein expression analysis

### Protein isolation

2-5 million cells were harvested by trypsinization, counted and washed twice with PBS. Cell pellets for total protein isolation were thoroughly lysed on ice with a 30-gauge insulin syringe (Omnican, Braun) in ice-cold protein lysis buffer supplemented with 0.1 mM of phenylmethylsulfonyl fluoride (PMSF, Sigma-Aldrich) and 1X protease inhibitors cocktail (Sigma-Aldrich). Protein lysis buffer was composed of 150 mM NaCl (Sigma-Aldrich), 50 mM Tris-HCl (pH 7.5, Sigma-Aldrich), 5 mM EDTA (pH 8, Sigma-Aldrich), 0.1 % (w/v) Sodium Dodecyl Sulfate (SDS, Sigma-Aldrich), 0.5 % (v/v) Nonidet P-40 (Sigma-Aldrich) and 0.5 % (v/v) N-Lauroylsarcosine (Sigma-Aldrich).

Once isolated, protein concentration was quantitated using a Nanodrop ND-1000 (Thermo Fisher Scientific) or by Bradford assay. For this procedure, a standard calibration curve of 7 known BSA concentrations was prepared. Then, 10 µL of the protein extracts and the calibration curve were loaded into a 96 well microtiter plate (Corning) and 300 µL of Bradford reagent (BioRad), previously diluted 1:5 in distilled water, were added. After incubating the mix for 5 min at 37 °C, absorbance was measured at 620 nm using a NanoQuant microplate reader Infinite M200 (Tecan) and protein concentration was extrapolated from the BSA standard curve.

### Western blot

Protein extracts were mixed with loading buffer, which consisted of 10 % (v/v) β-mercaptoethanol (Sigma-Aldrich), 10 % SDS, 50 % (v/v) glycerol, 200 mM Tris-HCl pH 6.8 and 0.1 % bromophenol blue (Sigma-Aldrich). They were boiled at 95 °C for 8 min and 20-30 µg protein were loaded into 8, 10 or 15 % acrylamide/bis-acrylamide self-made gels, depending on the molecular weight of the target protein/s to investigate. Gels were run at 80 V for approximately 2 h using a Mini-PROTEAN Tetra electrophoresis system (BioRad), and then transferred to methanol-preactivated polyvinylidene fluoride (PVDF) membranes (Millipore) at 100 V and 4 °C for 1-1.5 h with a Mini Trans-Blot Electrophoretic Transfer Cell (BioRad). Afterwards, membranes with the transferred proteins were stained with ATX Ponceau S red staining solution (Sigma-Aldrich) for 10 min at room temperature, photographed in an Amersham Imager 600 (GE Healthcare) and unstained with distilled water. They were then blocked for 1 h at room temperature with 5 % skim milk (Sigma-Aldrich) diluted in TBST buffer (Tris-buffered saline TBS composed of 20 mM Tris and 150 mM NaCl, supplemented with 0.05 % (v/v) Tween-20, Sigma-Aldrich) with shaking. Next, membranes were incubated overnight at 4 °C with rotation with primary antibodies of interest (see antibody references in **Table 3**), diluted in 5 % skim milk in TBST. After 4-5 TBST washes (5 min/wash), membranes were incubated for 1 h at room temperature with rotation with secondary antibodies conjugated to horseradish peroxidase (HRP) (see antibody references in **Table 3**), diluted in 5 % skim milk in TBST. Membranes were rinsed again 4-5 times (5 min/wash) with TBST and the signal was developed with Pierce ECL Western Blotting Substrate (Thermo Fisher Scientific), in either a LAS-3000 Imager (Fujifilm) or an Amersham Imager 600 (GE Healthcare). When necessary, membranes were stripped for 15 min with a pH 2.2 buffer composed of 7.5 glycine, 0.5 g SDS and 5 mL Tween20 in 500 mL distilled water. Once stripped, membranes were washed with TBST for 15 min, re-blocked with 5 % skim milk and re-probed with the antibodies of choice. Ponceau staining and western blot images were analysed and their band intensities quantitated with Image J software.

### Example 1.6. Proliferation assay

Proliferation of tumour cell lines was evaluated using the Cell Counting Kit-8 (Dojindo Molecular Technologies). This kit comprises WST-8, a highly water-soluble tetrazolium salt, which upon reduction by cellular dehydrogenases of viable cells, gives rise to a yellow formazan dye that is solube in cell culture media and can be quantitated. Briefly, similar number of cells (1 000-5 000) were plated in 96-well plates (Corning) and left to grow for the desired time period under different treatment conditions. Then, medium was renewed (100 µL volume/well) and 10 µL of CCK-8 reagent were added to each well. Plates were incubated in the dark for 3 h at 37 °C, and absorbance was measured at 450 nm in a NanoQuant Infinite 200 plate reader (Tecan).

### Example 1.7. Colony formation assay

For assessing the clonogenic capacity of tumour cells lines, they were plated as single-cell suspensions (5 000-10 000 cells) in 6 or 12-well plates (Corning) and incubated for 6-7 days with media change every 48 h. After this time, they were washed with PBS, fixed with 4 % PFA for 15 min at room temperature and stained with 0.1 % crystal violet dye (Sigma-Aldrich) for 20 min at room temperature. They were then rinsed with PBS, air dried and photographed. The staining was dissolved with 30 % acetic acid for 10 min on a shaker. The eluent was then transferred to a 96-well plate and the absorbance, indicative of colony growth, measured at 570 nm using a NanoQuant microplate reader Infinite M200 (Tecan).

### Example 1.8. Intracellular reactive oxygen species (ROS) measurement

For the determination of intracellular ROS production in HCC1937 cells subjected to olaparib treatment, 4 000 (EV_HCC1937) and 10 000 (Cx43_HCC1937) cells were plated in duplicate in a 96 well black polystyrene microplate (Corning) and treated with 50 and 100 µM olaparib for 96 h with media change at 48 h. Then, ROS production was determined using the DCFDA/H2DCFDA - Cellular ROS Assay Kit (Abcam) following manufacturer's instructions. Briefly, after 96 h of olaparib treatment, positive control cells were treated with 100 µM tert-butyl hydroperoxide (TBHP) solution prepared in phenol red-free DMEM (Lonza) with 10 % FBS for 2 h. Afterwards, all cell conditions were washed with 100 µL/well of 1X Buffer and then stained with 100 µL/well of 25 µM 2',7' -dichlorofluorescin diacetate (DCFDA) working solution for 45 min at 37 °C in the dark. DCFDA is a fluorogenic dye, deacetylated by cellular esterases to a non-fluorescent compound, eventually oxidized by ROS into the highly fluorescent 2', 7' -dichlorofluorescein (DCF). DCFDA solution was removed and cells were washed once with phenol red-free DMEM with 10 % FBS. They were left for 1 h at 37 °C and fluorescence was measured in a Synergy HTX plate reader (BioTek) at 485/+-20 nm (excitation) and 528/+-20 nm (emission).

After ROS measurement, CCK-8 proliferation assay was performed. Briefly, medium was removed and replenished with 100 µL/well of complete RPMI. 10 µL of CCK-8 reagent were added to each well, and cells were incubated at 37 °C in the dark for 3 h. Absorbance was measured at 450 nm in a NanoQuant Infinite 200 plate reader (Tecan).

Background fluorescence of phenol red-free DMEM with 10 % FBS was subtracted from all ROS fluorescence values, which were then normalized against their respective proliferation absorbance values. Each experimental condition was further normalized against its untreated counterpart.

### Example 1.9. Cell culture under Anoikis-inducing conditions

HCC1937 cells were cultured under anchorage-independent growth conditions for 48 h. For this purpose, non-adherent 90 mm polystyrene Petri dishes (Thermo Fisher Scientific) were coated with 10 mg/ml poly-HEMA (Sigma-Aldrich), which makes the dish surface highly repellent to cells, promoting their culture under forced-suspension conditions. 1-3 million cells were then directly plated in the presence/absence of 10 % FBS and olaparib 50 µM and left unperturbed. Images of the cultures were taken at 0 and 48 h, and cell pellets were then collected for western blot analysis.

### Example 1.10. Three-dimensional tumour spheroids

The bottom of 96-well flat bottom microplates (Greiner Bio-One) was coated with 1.5 % solution of UltraPure Low Melting Point Agarose (Invitrogen, Thermo Fisher Scientific). Agarose concave bottoms provide non-adhesive conditions and increase cell contacts. 100-200 µL of single-cell suspensions were plated on top of the agarose diluted in their respective media supplemented with 3.5 % Matrigel Matrix Basement Membrane Growth Factor Reduced (Corning) and 5 ng/mL human recombinant EGF (Sigma-Aldrich). Afterwards, plates were incubated under standard cell culture conditions and spheroids were monitored from day 2 onwards by optical microscopy.

### Example 1.11. Small extracellular vesicle (sEV) isolation and analysis

For small extracellular vesicle (sEV) isolation, sEV donor cells were grown in 175 cm² flasks (Thermo Fisher Scientific) until 85 % confluence. They were then carefully washed with sterile PBS three times and cultured in FBS-deprived media for 48 h. sEV-containing supernatants were collected, centrifuged at 1800 *x* g and filtered through a 0.22 µm filter to discard any cells. Donor cells final density was calculated, and supernatants derived from the same number of cells were ultracentrifuged at 100 000 *x* g for 90 min at 4 °C using a Hitachi Ultracentrifuge CP100NX with a P70AT rotor (Hitachi). After discarding the supernatant, the sEV-containing pellets were collected. For western blot analysis, pellets were ultracentrifuged again at 100 000 *x* g for 90 min at 4 °C in PBS, and then lysed in protein lysis buffer. If sEVs were to be used for *in vitro* treatments, pellets were directly collected in the appropriate cell culture media, combined with the pertinent drug if applicable, incubated at 37 °C for 1 h and eventually added to the target cells. *In vitro* treatments involving sEVs were renewed every 48 h with freshly isolated sEVs. sEVs for western blot or *in vitro* treatments were usually isolated from 1 175 cm² flask of donor cells, unless specified otherwise.

### Example 1.12. 2D and 3D migration assays

To assess the capability of tumour cells to migrate in 2D, scratch wound healing assay was performed coupled to time-lapse live cell imaging using a CQ1 confocal quantitative image cytometer (Yokogawa) over the course of 14 h. This experiment was performed under physiological conditions, at 37 °C and 5 % CO2 in a humidified environment. Cells were grown until 95 % confluency and serum-starved overnight. The cellular monolayer was then scratched using a 200 µL pipette tip angled at 30° and FBS was reduced in the medium from 10 to 0.2 %. Cells were then put inside the cytometer and images were taken at times 0, 4, 7, 10 and 14 h-post scratch. The scratch coverage (cell-free area) at 0, 7 and 14 h was quantitated using Wound_healing_size_tool Image J plugin (Suarez-Arnedo et al., 2020), and represented as a fold of time 0 h.

For spheroid-based migration assay, spheroids were generated under ultra-low attrachment conditions, and 72 h-post plating, their media was changed for one containing 2 % FBS and they were transferred to the inner wells of a 0.1 % gelatin-coated (Sigma-Aldrich) 96-well flat bottom microplate (Corning). Spheroids were centered in each well of the plate and left to adhere for 3 h at 37 °C. They were then individually photographed (t=0 h) under an inverted Nikon Eclipse Ti microscope (Nikon) and incubated for 24 h under standard cell culture conditions. After 24 h, they were imaged again, and the migration areas of individual spheroids at times 0 and 24 h were measured using Breast Analyser software. 24 h migration rate was then expressed as a fold of the area occupied by the spheroid at time 0 h.

### Example 1.13. Statistical analysis

Statistical analysis of data was performed using GraphPad Prism software version 8.0.2. Results are presented as mean ± standard deviation (SD) or standard error of the mean (SEM). For comparing two groups, two-tailed Student's t-test was carried out. Regarding evaluation of more than two groups, multiple t-tests or one-way analysis of variance (ANOVA) were used. Extra sum-of-squares F test was performed to examine differences in best-fit parameter logIC50 among tumour cell lines treated with different drugs.

Statistical differences were considered significant at a P < 0.05, with *P<0.05, **P<0.01, ***P<0.001, ns=not significant. Most experiments were performed at least 3 independent times (n=3), unless specified, generally including at least two replicates per n.

### Example 2. Results

### Example 2.1. Cx43 significantly resensitizes BRCA1 mutated triple negative breast cancer cells to the PARP inhibitor olaparib.

Cx43 effects on the tumoral response to the PARPi olaparib were studied, in order to determine whether its restoration could provide an additional benefit besides suppressing the tumoral processes.

Dose-response curves of HCC1937 treated with the PARP inhibitor olaparib (0-1200 µM) and its corresponding solvent DMSO were also obtained (**Figure 1****.A**). Cell viability was relativized to untreated controls for each cell line. Cx43 overexpression (green) was seen to provide a significantly advantageous therapeutic response in olaparib-treated HCC1937 cells at all concentrations tested until 1000 µM, and this was not affected by olaparib dissolution in the solvent DMSO until 600 µM, far above the drug concentrations used in this study. Olaparib IC50 was 601.9 µM for control empty vector (EV) and 350.1 µM for Cx43_HCC1937 (**Figure 1****.A**).

Next, proliferation assays with intermediate olaparib concentrations of 100 and 200 µM were performed with HCC1937 cells over the course of 96 h (**Figure 1****.B**). Cell viability was either relativized to untreated controls for each cell line at each time point, in order to account for the already reduced proliferation of Cx43-restituted cells (top graph) or reported as raw absorbance value (bottom graph). A robust and significant decrease in cell viability was observed in Cx43_HCC1937 cells (white) treated with olaparib when compared to EV_HCC1937 cells (grey), maintained at all concentrations and time points (**Figure 1****.B**).

These previous results were further confirmed with colony formation assays of HCC1937 cells treated with 100 µM olaparib over the course of seven days (**Figure 1****.C**). Cell viability measures were either relativized to untreated controls for each cell line, in order to account for the already reduced proliferation of Cx43-restituted cells (left graph) or reported as raw absorbance values (right graph). Treatment with 100 µM olaparib led to a profound decrease in HCC1937 cell viability upon Cx43 restitution (**Figure 1****.C**).

### Example 2.2. Cx43 reduces global protein PARylation and is associated with lower levels of RAD51 in BRCA1 mutated triple negative breast cancer cells under olaparib treatment

In order to establish PARP functionality in HCC1937 during olaparib treatment, total poly(ADP-ribose) (PAR) polymer, the product of PARP-1 activity, was assessed by western blot in olaparib-treated (50/100 µM) HCC1937 cells for 48 and 96 h (**Figure 2****.A**). Over the course of time and the increase in treatment concentration, it could be seen how PAR levels rose in EV_HCC1937cells, but decreased in Cx43_HCC1937, in line with a lack of response and a positive response to PARP inhibition, respectively. PAR distribution was also examined by immunofluorescence in HCC1937 cells treated with olaparib 100 µM for 96 h (**Figure 2****.B**). A similar trend as that observed in **Figure 2****.A** could be seen here: PAR (red) significantly increased in EV_HCC1937 cells after olaparib treatment, while it decreased in Cx43_HCC1937, with the peculiarity of a mostly nuclear signal in untreated EV_HCC1937 and cytoplasmic in Cx43_HCC1937 (**Figure 2****.B**). Variations in PAR localization can be attributed to cell cycle differences, with higher nuclear-to-cytoplasmic PAR ratios during S phase, and lower in G0/G1 stages.

In line with this line of research, RAD51 and 53BP1, involved in DNA double-strand breaks (DSBs) repair by homologous recombination (HR) and nonhomologous end joining (NHEJ), were assessed by immunofluorescence in HCC1937 cells treated with olaparib 100 µM for 96 h (**Figure 2****.C**). RAD51 has been positively linked to PARPi resistance, whereas loss of 53BP1 is associated with less sensitivity to PARPi. 53BP1 (green) foci seemed to rise upon olaparib treatment in both cell lines alike. However, RAD51 (red) foci were increased just in EV_HCC1937 cells treated with the drug, with only a few Cx43_HCC1937 cells positive for RAD51, hinting at a possible mechanism of resistance in HCC1937 EV cells (**Figure 2****.C**).

### Example 2.3. Cx43 significantly increases intracellular reactive oxygen species (ROS) production in BRCA1 mutated triple negative breast cancer cells during olaparib treatment

Next, intracellular reactive oxygen species (ROS) production in HCC1937 pIRES cells was investigated after treatment with olaparib 50 and 100 µM for 96 h (**Figure 3**). Positive control cells were treated the day of the measurement with the ROS inducer tert-butyl hydroperoxide (TBHP) at 100 µM for 2 h. After treatment with the compounds, all cells were stained with 2',7'-dichlorofluorescin diacetate (DCFDA), a fluorogenic dye that produces the highly fluorescent compound 2',7'-dichlorofluorescein (DCF) upon oxidation by cellular ROS, and is detected by fluorescence spectroscopy (485 nm excitation, 535 nm emission). After ROS measurement, the cell density was quantitated, and ROS production fluorescence values for each condition were normalized against their respective proliferation values (left graph) to account for different cell densities. Each experimental condition was further normalized against its untreated counterpart (right graph). No statistically significant differences were observed among untreated EV_HCC1937 (grey) and Cx43_HCC1937 (white) cells, nor between positive TBHP-treated controls. Upon treatment with olaparib, Cx43_HCC1937 ROS production increased whereas it diminished in EV_HCC1937 in a dose-dependent manner (left graph). Overall ROS values were significantly higher in olaparib-treated Cx43_HCC1937 than in their EV counterparts (right graph), which can potentially be associated with DNA damage accumulation and elevated apoptotic response to olaparib in Cx43_HCC1937, as will be seen in **Figure 4****.B-C** and **Figure 5****.A.**

Increased ROS levels are considered a double-edged sword in cancer, as they can promote tumour progression by affecting the microenvironment but are also related to reduced multidrug resistance and increased cell death, particularly by apoptosis, autophagy and necroptosis. Recent work also points to a cytotoxic effect (DNA damage) of mitochondria-derived elevated ROS levels resulting from olaparib treatment.

### Example 2.4. Olaparib significantly increases p21-mediated senescence and DNA damage accumulation in Cx43-restituted BRCA1 mutated triple negative breast cancer cells

Cellular senescence is a process usually considered as cancer suppressive due to its involvement in the prevention of tumour cell proliferation and progression, and its effector role in several antitumoral treatments. As olaparib therapy has been described to promote senescence via increased p16, increased p53 or increased RB806 and decreased p53, depending on the ovarian cancer cell type, senescence was studied by western blot in olaparib-treated (50/100 µM) HCC1937 cells for 48 and 96 h (**Figure 4****.A**). The senescence marker p21 was significantly increased in Cx43_HCC1937 after 96 h 100 µM olaparib treatment, which in turn had significantly higher levels than their 96 h 100 µM olaparib-treated EV_HCC1937 cells.

Finally, taking into account olaparib mechanism of action, γH2AX (p-Ser139), a marker of DNA damage (DNA double-strand breaks - dsDNA breaks) and cellular senescence, was assessed by western blot, in olaparib-treated (50/100 µM) HCC1937 cells for 48 and 96 h (**Figure 4****.B**). 50/100 µM olaparib 96 h treatment in Cx43_HCC1937 cells led to a significant γH2AX increase when compared to their basal condition and the same treatments in EV_HCC1937 cells, indicating a greater accumulation without repair of dsDNA breaks. γH2AX was also assessed by immunofluorescence in HCC1937 treated with olaparib 50 and 100 µM for 96 h (**Figure 4****.C**). Untreated Cx43_HCC1937 cells showed already a higher number of γH2AX foci (green) than EV_HCC1937, and this trend was increased during olaparib treatment, supporting the fact that olaparib leads to more dsDNA damage accumulation in Cx43_HCC1937 (**Figure 4****.C**).

### Example 2.5. Cx43 significantly increases programmed cell death in BRCA1 mutated triple negative breast cancer cells under olaparib treatment

To further characterize the effect of Cx43 restitution in resensitizing HCC1937 to olaparib, western blots were performed against several cell death-associated proteins in olaparib-treated (50/100 µM) HCC1937 cells for 48 and 96 h (**Figure 5**). Cx43_HCC1937 showed a significantly robust increase in active cleaved caspase 3 after 96 h of 50 and 100 µM olaparib treatment, as opposed to almost no response in EV_HCC1937. PARP-1 and PARP-2 had no significant changes in overall levels among Cx43 and EV_HCC1937 in any of the conditions; strong PARP-1 cleavage was detected in Cx43_HCC1937 cells after after 96 h of 50 and 100 µM olaparib treatment, as opposed to low cleavage levels in EV_HCC1937. These results suggest increased apoptosis in Cx43_HCC1937 cells after olaparib treatment via cleaved caspase 3, which likely induces downstream PARP-1 cleavage. Cleaved PARP-1 presumably further inhibits the catalytic activity of the still uncleaved protein, allowing conservation of its substrate NAD+ and as result of ATP, enabling cells to conduct programmed cell death.

### Example 2.6. Cx43 significantly increases programmed cell death and DNA damage and reduces global PARylation in BRCA1 mutated triple negative breast tumour cells during olaparib treatment under Anoikis-inducing conditions.

In order to test the effect of Cx43 on the tumoral response to olaparib in a more physiologically relevant setting, HCC1937 cells were treated with 50 µM olaparib and cultured under Anoikis-inducing anchorage-independent/forced suspension conditions for 48 h (**Figure 6****.A**).

Anoikis, a term first coined in 1994, refers to a form of programmed cell death induced by disruption of cell adhesion of normal epithelial cells that detach from the surrounding extracellular matrix. Anoikis resistance has been identified as a hallmark of cancer cells, as survival of normally adherent cells in suspension conditions can enable them to metastasize in distant sites. It has been studied both in the presence and absence of serum; serum deprivation constitutes another way of measuring cell resilience under adverse circumstances, as fetal bovine serum (FBS) contains a variety of growth factors and other elements required for cell survival in culture.

Under these circumstances, and as opposed to the results observed after 48 h of regular culture circumstances (**Figure 5**), cleaved caspase 3 levels were already significantly higher in Cx43_HCC1937 cells after only 48 h of Anoikis induction, particularly in the absence of serum (**Figure 6****.A**). Olaparib treatment of Cx43_HCC1937 cells further increased this cleavage, even more when serum was deprived, and contrasted with no significant changes in their EV counterparts. Regarding γH2AX, untreated Cx43_HCC1937 showed already significantly higher levels than their EV counterparts, being the effect more pronounced when serum was removed (**Figure 6****.A**). Olaparib treatment in the absence of serum further exacerbated this outcome (**Figure 6****.A**), as also seen in normal culture conditions (**Figure 4****.B**). Total protein PARylation, directly resulting from PARP activity, seemed to also follow the trend observed under regular culture settings (**Figure 2****.A-B**), with a rise in EV_HCC1937 cells, but a decrease in Cx43_HCC1937, after olaparib treatment (**Figure 6****.A**), indicating effective inhibition of PARP enzymatic activity only upon Cx43 restoration.

These experiments suggest not only a confirmation in Anoikis-inducing conditions of the results observed under normal culture circumstances (**Figure 2****,** **Figure 4** & **Figure 5**), but also an increased apoptotic effect. Untreated suspension-forced, particularly serum-deprived, Cx43_HCC1937 cells were already more sensitive to Anoikis than their EV counterparts (Cx43 restitution provides Anoikis resensitization), so 48 h of 50 µM olaparib treatment in these conditions led to higher Anoikis/apoptotic response (**Figure 6****.A**) in Cx43_HCC1937 cells without having to wait 96 h for a similar effect under normal culture settings (**Figure 5**), where hardly no apoptotic response was detected at the 48 h time point.

In line with these results, under 48 h Anoikis-inducing conditions HCC1937 cells grew in suspension and tended to form aggregates, appearing larger in EV_HCC1937 than in Cx43_HCC1937, and generally smaller in the absence of serum (**Figure 6****.B**). 50 µM olaparib treatment resulted in the partial disintegration of these clusters, leaving a higher percentage of loose floating cells, part of which were presumably dead according to **Figure 6****.A;** this effect was more pronounced in Cx43_HCC1937 than in its EV counterpart, indicating an ability of wild type HCC1937 cells to survive in suspension conditions and a resensitization of HCC1937 cells to Anoikis and to olaparib upon Cx43 restoration.

### Example 2.7. Olaparib treatment in Cx43-restituted BRCA1 mutated triple negative breast cancer cells leads to significantly lower 3D spheroid proliferation than in wild type cells

In order to further test the potential of Cx43 to increase olaparib effect in HCC1937 cells in preclinical models involving a more complex and heterogeneous environment, representative of in vivo tumours, three-dimensional EV and Cx43_HCC1937 spheroids were obtained (**Figure** 7).

Uniform-sized spheroids were produced by plating 3000 EV and Cx43_HCC1937 cells under ultra-low attachment conditions, as well as 5000 Cx43_HCC1937 cells in parallel to account for the expected anti-proliferative effect of Cx43 in untreated tumour cells (day -4) (**Figure 7**). When spheroids were 4 days old and of somewhat similar initial size (comparing EV_HCC1937 spheroids plated at 3000 cells with Cx43_HCC1937 spheroids plated at 5000 cell density), olaparib treatment (200, 400 and 600 µM) started (day 0 treatment), for a total period of 8 days. Spheroids were individually tracked during this time and their size was registered every 48 h. Olaparib concentrations were higher than those used for 2D experiments to account for the expected higher drug resistance in 3D models.

Afterwards, spheroid area measurements were pooled for each condition and EV spheroids were compared against Cx43_HCC1937 spheroids, either those plated at 3000 or 5000 initial cell densities. In all four conditions (untreated, olaparib 200, 400 and 600 µM), Cx43_HCC1937 spheroids (green) plated at the same initial cell density as EV (red) spheroids (3000 cells), were significantly smaller. After 8 days, these Cx43-restituted 3D models were on average 41.7, 29.68 and 12.57 % smaller than their EV counterparts when under olaparib 200, 400 and 600 µM treatment, respectively.

Regarding Cx43_HCC1937 spheroids plated at 5000 initial cell density (blue), they were also significantly smaller than EV_HCC1937, particularly at the later stages of the treatment (days 4, 6 and 8), even when plated at almost twice the initial cell density. Whereas untreated EV_HCC1937 spheroids increased their size along time, Cx43_HCC1937 did not, instead leading to a slight decrease in their initial size (day 0), either due to increased cell death or enhanced tightness. It is interesting to note that after 8 days of 600 µM olaparib treatment, Cx43_HCC1937 spheroids (initial cell densities 3000 and 5000) were almost non-existent, compared to still well-defined EV_HCC1937 spheroids (**Figure 7**).

These results further reinforce in a preclinical model the potential therapeutic advantage of Cx43-restituted *BRCA1* mutated tumours under PARPi olaparib treatment.

### Example 2.8. Cx43 restitution significantly resensitizes BRCA1 mutated ovarian cancer cell line SNU251 to olaparib treatment

In order to complement previous results with another clinically relevant PARPi-treated cancer, assays were repeated with *BRCA1* mutated ovarian cancer cell line SNU251, efficiently nucleofected with EV or Cx43-restituting plasmids (**Figure 8**). SNU251 wild type (EV) cells had no native Cx43 expression, as assayed by western blot ( **Figure 8****.A**).

Next, proliferation assays were performed for 96 h in the presence of 100 and 200 µM olaparib (**Figure 8****.B**). Cx43-restored SNU251 cells (white) showed a significantly better response to olaparib than EV cells (grey), beginning at 48 h, both with normalized (left graph) and raw proliferation data (right graph), although the difference was more acute in the latter.

So far, breast HCC1937 and ovarian SNU251 *BRCA1* mutated cells can be efficiently resensitized to olaparib treatment upon Cx43 restitution (**Figure 1** **-** **Figure 7** and **Figure 8**).

### Example 2.9. Small extracellular vesicles (sEVs) containing Cx43 improve olaparib efficacy in de novo resistant BRCA1 mutated HCC1937 cells

Small extracellular vesicles (sEVs) are cell-derived phospholipid-based nanoparticles that allow near and distant cell-cell communication and are implicated in different physiological and pathological processes, and more recently have garnered great interest as promising next-generation nanocarriers for the targeted delivery of various therapeutics. Cx43 has been determined to be present in certain sEVs, playing a key role in facilitating their cargo delivery and uptake by target cells.

In order to assess the therapeutic potential of sEVs as Cx43 carriers/delivery vehicles in combination with olaparib, wild type HCC1937 cells, which are known to be *de novo* resistant to olaparib, were treated with Cx43-null and Cx43-enriched sEVs isolated from different cell sources: the human embryonic kidney HEK-293T cell line, a widely used source of sEVs in clinical trials due to its various advantages, and HCC1937 control (EV) and Cx43-restituted cells (autologous sEVs). Both (i) sEVs derived from non-tumoral cells (such as HEK-293T) and (ii) Cx43_HCC1937-autologous sEVs contain high Cx43 levels, whereas EV_HCC1937-sEVs do not contain Cx43; all sEVs stained positive for the tetraspanin proteins CD9 and CD63, which are sEVs markers (**Figure 9****.A**). Five-day treatment with Cx43-positive sEVs isolated from two different cell sources (HEK-293T-sEVS, named "sEVs-Cx43+" in the figure, and Cx43_HCC1937-sEVs, named "Cx43+_HCC", highlighted with red squares) significantly improved 75 µM olaparib cytotoxic effect versus olaparib alone in HCC1937 cells, as determined by colony forming assay (**Figure 9****.B**), supporting their potential application in pre/clinical settings for the targeted delivery of this drug.

### Example 2.10. Cx43 acts as a tumour suppressor in BRCA1/2 mutated cancer

To further characterize its involvement in *BRCA1*/*2* mutated cancer, Cx43 was restituted by plasmid transfection in *BRCA1* mutated TNBC and ovarian carcinoma cell lines, HCC1937 and SNU251, respectively. Proliferation experiments over the course of 96 h showed a significant reduction in proliferative activity of Cx43-restituted HCC1937 and SNU251 cells (green/white) when compared with control EV cells (grey) (**Figure 10****.A** left and center). These results were further confirmed by colony formation assays in HCC1937 cells after 6 days in culture (**Figure 10****.A** left), corroborating that Cx43 restoration significantly reduced colony formation when compared to the EV counterparts. Similarly, *BRCA2* mutated lung cancer Ludlu-1 wild type cells were treated for 5 days with Cx43-enriched sEVs (Cx43-sEVs), acting as Cx43 delivery vehicles and derived from non-tumoral HEK293T cells, leading to a reduction in tumour cell proliferation (**Figure 10****.A** right). The combination of Cx43-sEVs with olaparib 75 µM also remarkedly enhanced the cytotoxic effect of the drug (**Figure 10****.A** right).

Next, Cx43 involvement in the TNBC pathogenesis was studied under more physiological approaches, namely 3D spheroid culture (**Figure 10****.B**) and Anoikis-inducing conditions (**Figure 10****.C**). 3D spheroid culture (**Figure 10****.B**) of HCC1937 cells for 12 days further corroborated the reduction in proliferation determined in 2D culture (**Figure 10****.A**) resulting from Cx43 restoration. Cx43_HCC1937 spheroids (green line) were significantly smaller than their EV counterparts (red line) at each time point measured (4, 6, 8, 10 and 12 days) when plated at the same initial cell density (3000 cells); interestingly, even if Cx43_HCC1937 cells (blue line) were plated at a higher cell density than EV_HCC1937 cells (red line) (5000 versus 3000 cells, respectively), their proliferation was still significantly slower and spheroids ended up turning smaller than their EV counterparts (**Figure 10****.B**).

Anoikis refers to a form of programmed cell death induced by disruption of cell adhesion of normal epithelial cells that detach from the surrounding extracellular matrix. Anoikis resistance has been identified as a hallmark of cancer cells, as survival of normally adherent cells in suspension conditions can enable them to metastasize to distant sites. It has been studied both in the presence and absence of serum; serum deprivation constitutes another way of measuring cell resilience under adverse circumstances, as fetal bovine serum (FBS) contains a variety of growth factors and other elements required for cell survival in culture. When cultured under anchorage-independent/forced suspension conditions for 48 h (**Figure 10****.C**), Cx43_HCC1937 cells formed smaller multicellular aggregates (dashed red lines) than EV_HCC1937, particularly in the absence of FBS. As assessed by western blot, cleavage of the apoptosis mediator caspase 3 was significantly increased in Cx43_HCC1937, especially under serum deprivation, as opposed to EV_HCC1937. Vimentin, a key contributor of epithelial-mesenchymal transition, was upregulated in EV_HCC1937 at all conditions, in line with Anoikis resistance, and deeply downregulated in Cx43_HCC11937 in a statistically significant manner. These experiments suggest Anoikis resensitization of HCC1937 upon Cx43 restitution, particularly when they are serum-deprived.

Finally, cellular migration of HCC1937 cells was studied under 2D (**Figure 10****.D**) and 3D-spheroid (**Figure 10****.E**) conditions. HCC1937 2D migration was determined by scratch/wound healing assay coupled with time-lapse microscopy under serum deprivation conditions for 14 h after the scratch of a cell monolayer (**Figure 10****.D**). The scratch coverage (cell-free area) at 0, 7 and 14 h was quantitated and expressed as a fold of the initial (time 0 h) wound area. Cx43-restituted HCC1937 cells (green) showed significantly more cell-free scratched area than their EV counterparts (black), indicating reduced migratory capacity (**Figure 10****.D**). Spheroid migration experiments were performed by separately culturing 3-day spheroids and plating them in gelatin-coated flat bottom microplates, assessing the migration after 24 h (**Figure 10****.E**; migration front indicated as dashed red lines). Cx43 restitution in HCC1937 led to significantly reduced spheroid migration (green) compared to its EV counterpart (red).

## Claims

1. Connexin 43 for use in the treatment of a *BRCA1*/*2* mutated cancer selected from: *BRCA1*/*2* mutated triple negative breast cancer (TNBC), *BRCA1*/*2* mutated ovarian cancer and/or *BRCA1*/*2* mutated lung cancer; wherein the delivery vehicle is a nanoparticle, an extracellular vesicle or an expression vector which encodes Connexin 43.

2. Connexin 43 for use, according to claim 1, wherein connexin 43 is administered concomitantly with a poly ADP ribose polymerase (PARP) inhibitor.

3. Connexin 43 for use, according to any of the previous claims, wherein connexin 43 is administered concomitantly with a PARP inhibitor selected from: Olaparib, niraparib, veliparib, talazoparib, iniparib or rucaparib.

4. Combination drug product comprising Connexin 43 and a PARP inhibitor; wherein the delivery vehicle is a nanoparticle, an extracellular vesicle or an expression vector which encodes Connexin 43.

5. Combination drug product, according to claim 4, wherein Connexin 43 can be selected between Connexin 43 protein, mRNA or DNA.

6. Combination drug product, according to any of the claims 4 or 5, comprising Connexin 43 and a PARP inhibitor selected from: Olaparib, niraparib, veliparib, talazoparib, iniparib or rucaparib.

7. Combination drug product, according to any of the claims 4 to 6, comprising Connexin 43 and olaparib.

8. Pharmaceutical composition comprising the combination drug product of any of the claims 4 to 7 and, optionally, pharmaceutically acceptable excipients or carriers; wherein the delivery vehicle is a nanoparticle, an extracellular vesicle or an expression vector which encodes Connexin 43.

9. Pharmaceutical composition, according to claim 8, for use in the treatment of a *BRCA1*/*2* mutated cancer selected from: *BRCA1*/*2* mutated TNBC, *BRCA1*/*2* mutated ovarian cancer and/or *BRCA1*/*2* mutated lung cancer; wherein the delivery vehicle is a nanoparticle, an extracellular vesicle or an expression vector which encodes Connexin 43.

## Patentansprüche

1. Connexin 43 zur Verwendung bei der Behandlung von einem *BRCA1*/*2*-mutierten Krebs ausgewählt aus: *BRCA1*/*2*-mutiertem dreifach negativem Brustkrebs (TNBC), *BRCA1*/*2*-mutiertem Eierstockkrebs und/oder *BRCA1*/*2*-mutiertem Lungenkrebs; wobei das Transportvehikel eine Nanopartikel, eine extrazelluläre Vesikel oder ein Expressionsvektor, welcher Connexin 43 kodiert, ist.

2. Connexin 43 zur Verwendung nach Anspruch 1, wobei das Connexin 43 gleichzeitig mit einem Poly(ADP-Ribose)Polymerase (PARP)-Inhibitor verabreicht wird.

3. Connexin 43 zur Verwendung nach einem der vorhergehenden Ansprüche, wobei das Connexin 43 gleichzeitig mit einem PARP-Inhibitor verabreicht wird, ausgewählt aus: Olaparib, Niraparib, Veliparib, Talazoparib, Iniparib oder Rucaparib.

4. Kombinationsarzneimittel umfassend Connexin 43 und einen PARP-Inhibitor; wobei das Transportvehikel eine Nanopartikel, eine extrazelluläre Vesikel oder ein Expressionsvektor, welcher Connexin 43 kodiert, ist.

5. Kombinationsarzneimittel nach Anspruch 4, wobei das Connexin 43 zwischen Connexin 43-Protein, -mRNA oder -DNA ausgewählt werden kann.

6. Kombinationsarzneimittel nach einem der Ansprüche 4 oder 5, umfassend Connexin 43 und einen PARP-Inhibitor ausgewählt aus: Olaparib, Niraparib, Veliparib, Talazoparib, Iniparib oder Rucaparib.

7. Kombinationsarzneimittel nach einem der Ansprüche 4 bis 6, umfassend Connexin 43 und Olaparib.

8. Pharmazeutische Zusammensetzung umfassend das Kombinationsarzneimittel nach einem der Ansprüche 4 bis 7 und, wahlweise, pharmazeutisch akzeptable Hilfsstoffe oder Träger; wobei das Transportvehikel eine Nanopartikel, eine extrazelluläre Vesikel oder ein Expressionsvektor, welcher Connexin 43 kodiert, ist.

9. Pharmazeutische Zusammensetzung nach Anspruch 8, zur Verwendung bei der Behandlung von einem *BRCA1*/*2*-mutierten Krebs ausgewählt aus: *BRCA1*/*2*-mutiertem TNBC, *BRCA1*/*2*-mutiertem Eierstockkrebs und/oder *BRCA1*/*2*-mutiertem Lungenkrebs; wobei das Transportvehikel eine Nanopartikel, eine extrazelluläre Vesikel oder ein Expressionsvektor, welcher Connexin 43 kodiert, ist.

## Revendications

1. Connexine 43 pour son utilisation dans le traitement d'un cancer porteur d'une mutation BRCA1/2 choisi parmi : le cancer du sein triple négatif (TNBC) porteur d'une mutation BRCA1/2, le cancer de l'ovaire porteur d'une mutation BRCA1/2 et/ou le cancer du poumon porteur d'une mutation BRCA1/2 ; dans laquelle le véhicule d'administration est une nanoparticule, une vésicule extracellulaire ou un vecteur d'expression codant pour la connexine 43.

2. Connexine 43 pour son utilisation, selon la revendication 1, dans laquelle la connexine 43 est administrée de manière concomitante avec un inhibiteur de la poly(ADP-ribose) polymérase (PARP).

3. Connexine 43 pour son utilisation, selon l'une quelconque des revendications précédentes, dans laquelle la connexine 43 est administrée de manière concomitante avec un inhibiteur de PARP choisi parmi : l'olaparib, le niraparib, le veliparib, le talazoparib, l'iniparib ou le rucaparib.

4. Produit pharmaceutique de combinaison comprenant la connexine 43 et un inhibiteur de PARP ; dans lequel le véhicule d'administration est une nanoparticule, une vésicule extracellulaire ou un vecteur d'expression codant pour la connexine 43.

5. Produit pharmaceutique de combinaison, selon la revendication 4, dans lequel la connexine 43 peut être choisie parmi protéine, ARNm ou ADN de la connexine 43.

6. Produit pharmaceutique de combinaison, selon l'une quelconque des revendications 4 ou 5, comprenant la connexine 43 et un inhibiteur de PARP choisi parmi : l'olaparib, le niraparib, le veliparib, le talazoparib, le iniparib ou le rucaparib.

7. Produit pharmaceutique de combinaison, selon l'une quelconque des revendications 4 à 6, comprenant la connexine 43 et l'olaparib.

8. Composition pharmaceutique comprenant le produit pharmaceutique de combinaison selon l'une quelconque des revendications 4 à 7 et, optionnellement, des excipients ou des porteurs pharmaceutiquement acceptables ; dans laquelle le véhicule d'administration est une nanoparticule, une vésicule extracellulaire ou un vecteur d'expression codant pour la connexine 43.

9. Composition pharmaceutique, selon la revendication 8, pour son utilisation dans le traitement d'un cancer porteur d'une mutation BRCA1/2 choisi parmi : le TNBC porteur d'une mutation BRCA1/2, le cancer de l'ovaire porteur d'une mutation BRCA1/2 et/ou le cancer du poumon porteur d'une mutation BRCA1/2 ; dans laquelle le véhicule d'administration est une nanoparticule, une vésicule extracellulaire ou un vecteur d'expression codant pour la connexine 43.
